# EUROPÄISCHE PATENTSCHRIFT

(11) **EP 3 621 848 B1**
(45) Veröffentlichungstag und Bekanntmachung des Hinweises auf die Patenterteilung: **31.03.2021**
(21) Anmeldenummer: 18723665.8
(22) Anmeldetag: 24.04.2018
(51) Int. Cl.: B60Q 1/08, G09B 19/16

(54) **VERFAHREN UND SYSTEM ZUR STEUERUNG BZW. ZUM TRAINIEREN DES FAHRVERHALTENS EINES FAHRERS ÜBER DESSEN BLICKLENKUNG**
METHOD AND SYSTEM FOR CONTROLLING OR TRAINING THE DRIVING BEHAVIOR OF A DRIVER BY DIRECTING THE VIEW OF THE DRIVER
PROCÉDÉ ET SYSTÈME DE COMMANDE OU D'APPRENTISSAGE DU COMPORTEMENT ROUTIER D'UN CONDUCTEUR PAR L'INTERMÉDIAIRE DE LA DIRECTION DE REGARD DE CELUI-CI

(30) Priorität: 10.05.2017 AT 503822017
(43) Veröffentlichungstag der Anmeldung: 18.03.2020
(73) Patentinhaber: ZKW Group GmbH, 3250 Wieselburg (AT)
(72) Erfinder: HARTMANN, Peter, 3392 Schönbühel an der Donau (AT); GRÜNER, Markus, 3814 Aigen bei Raabs (AT); ANSORGE, Ulrich, 1170 Wien (AT); BÜSEL, Christian, 6020 Innsbruck (AT); BEDNAR, Ingeborg, 1130 Wien (AT); ALTMANN, Johann, 3950 Gmünd (AT)
(74) Vertreter: Patentanwaltskanzlei Matschnig & Forsthuber OG
(86) Internationale Anmeldenummer: PCT/AT2018/060077
(87) Internationale Veröffentlichungsnummer: WO 2018/204960

(56) Entgegenhaltungen:
- WO-A1-2015/006793
- DE-A1-102009 048 619
- WALDNER MANUELA ET AL: "Attractive Flicker - Guiding Attention in Dynamic Narrative Visualizat", IEEE TRANSACTIONS ON VISUALIZATION AND COMPUTER GRAPHICS, IEEE SERVICE CENTER, LOS ALAMITOS, CA, US, Bd. 20, Nr. 12, 31. Dezember 2014 (2014-12-31), Seiten 2456-2465, XP011563276, ISSN: 1077-2626, DOI: 10.1109/TVCG.2014.2346352 [gefunden am 2014-10-23] in der Anmeldung erwähnt
- LAURA POMARJANSCHI ET AL: "Gaze guidance reduces the number of collisions with pedestrians in a driving simulator", ACM TRANSACTIONS ON INTERACTIVE INTELLIGENT SYSTEMS (TIIS), Bd. 1, Nr. 2, 1. Januar 2012 (2012-01-01), Seiten 1-14, XP055416312, 2 Penn Plaza, Suite 701 New York NY 10121-0701 USA ISSN: 2160-6455, DOI: 10.1145/2070719.2070721

## Beschreibung

Die Erfindung betrifft ein Verfahren zur Steuerung und/oder zum Trainieren des Fahrverhaltens eines Fahrers beim Führen eines Kraftfahrzeugs, wobei das Kraftfahrzeug aufweist: (i) mindestens eine den Fahrer betreffende Blickerfassungseinrichtung, (ii) Scheinwerfermittel, die dazu eingerichtet sind, mindestens eine vorgebbare Lichtverteilung vor dem Kraftfahrzeug zu erzeugen, oder mindestens eine Frontleuchte, und (iii) Mittel zum Erfassen der aktuellen Verkehrssituation, wobei das Verfahren umfasst: kontinuierliches Erfassen zumindest eines Blickparameters des Fahrers mittels der mindestens einen Blickerfassungs-Einrichtung (Eyetrackingeinrichtung), wobei der Blickparameter aus der Gruppe bestehend aus einer Blickrichtung, einer Blickbewegung und einer Blickdauer des Fahrers sowie einer Kombination dieser Blickparameter ausgewählt ist, und zumindest ein Hinweissignal für den Fahrer ausgelöst wird, falls die erfassten Blickparameter und/oder die Verkehrssituation im Vergleich zu abgespeicherten diesbezüglichen Normalwerten Abweichungen über vorgebbare Grenzen hinaus aufweisen.

Die Erfindung betrifft außerdem ein Fahrassistenzsystem für ein Kraftfahrzeug zum Ausführen des Verfahrens zur Steuerung und/oder zum Trainieren des Fahrverhaltens eines Fahrers gemäß der Erfindung, umfassend(i) mindestens eine den Fahrer betreffende Blickerfassungs-Einrichtung (Eyetrackingeinrichtung), die zum kontinuierlichen Erfassen zumindest eines Blickparameters des Fahrers eingerichtet ist, wobei der Blickparameter aus der Gruppe bestehend aus einer Blickrichtung, einer Blickbewegung und/oder einer Blickdauer ausgewählt ist, (ii) Scheinwerfermittel, die dazu eingerichtet sind, zumindest eine vorgebbare Lichtverteilung vor dem Kraftfahrzeug zu erzeugen, oder mindestens eine Frontleuchte, (iii) Mittel zum Erfassen der aktuellen Verkehrssituation, und (iv) eine Steuerungs- und Auswerteeinrichtung, welche dazu eingerichtet ist, die Scheinwerfermittel oder die mindestens eine Frontleuchte zu steuern.

Ferner betrifft die Erfindung ein Kraftfahrzeug, welches ein erfindungsgemäßes Fahrassistenzsystem wie hierin beschrieben umfasst.

Es besteht nicht zwingend bzw. unter allen Umständen ein grundsätzlicher Zusammenhang zwischen mehr Licht auf der Straße, wie moderne Adaptive Frontlighting Systeme (AFS)-bzw. Adaptive Driving Beam (ADB)-Scheinwerfer heutzutage definitiv bieten, und einem Sicherheitszugewinn im Verkehr. Um dem volkswirtschaftlichen Ziel der Steigerung der Verkehrssicherheit einerseits sowie selbstverständlich auch einer betriebswirtschaftlich zielgerichteten Produktentwicklung im Spannungsfeld zwischen Kosten und aufwändigen, technisch riskanten Lösungsvarianten gerecht zu werden, ist ein tiefes Verständnis der Wirkung des Lichts auf die Wahrnehmung und in weiterer Folge das Verhalten des Fahrers von hoher, wenn nicht gar entscheidender Bedeutung, da erst mit diesem Verständnis sowie darauf basierenden Algorithmen zielgerichtet förderliche "Sichtsysteme" für Nachtfahrten ermöglicht werden.

Das Problem, warum mehr Ausleuchtung durch AFS- bzw. ADB-Scheinwerfer nicht unmittelbar mit einem direkten Sicherheitsgewinn einhergehen muss, ist grundlegender, kognitionspsychologischer Natur:
- Oft überlagern eine anders gelagerte Intention sowie der Erfahrungshintergrund eine aufgrund grundsätzlich verbesserter Erkennbarkeit mögliche Wahrnehmung von schwach kontrastierten Objekten, die ein Gefahrenmoment darstellen (könnten).
- Die durch moderne Scheinwerfertechniken eingebrachte Dynamik im Lichtbild (erkennbare Zu/ Abschaltung von Lichtsegmenten) wird oftmals als unangenehm und störend empfunden (Kontrastveränderung innerhalb der Lichtverteilung; sprunghafte oder kontinuierliche Lageänderung von Hell-Dunkel-Übergängen bei ADB-Scheinwerfern; Blitzer durch Eigenblendungen an Schildern oder Scheiben; störendes Flackern bei sensibel eingestellten Pixel-Licht-Systemen; Farbspiel bei abzubildenden Blendenkanten durch LED Linsenfehler) und kann auch von wesentlichen visuellen Erkennungsaufgaben ablenken.
- Adaptation: Mehr Ausleuchtung wird nicht immer als solche empfunden, da sich das Auge anpasst.
- Gewohnheit und Erfahrungen prägen sehr stark das Fahrverhalten, dieses wird nicht (immer) durch bessere Ausleuchtung verbessert, sondern kann auch tendenziell ins riskantere Verhalten verschoben werden.
- Ein gewisser "Kontrollverslust" über das Licht wird von manchen Fahrern als störend empfunden.

Verfahren und Vorrichtungen zur Beeinflussung der Blickrichtung oder zur Erregung der Aufmerksamkeit von menschlichen Individuen durch optische Reize sind aus dem Stand der Technik bekannt.

Die US20030181822 A1 offenbart ein System und ein Verfahren, um die visuelle Aufmerksamkeit eines Fahrers zu erkennen und ein Human Machine Interface (HMI)-Gerät zu aktivieren, um die Blickrichtung des Fahrers mittels optischen, gegebenenfalls subtilen, Blickreizen in eine bestimmte Richtung zu dirigieren (in der einschlägigen Fachliteratur auch als "gaze redirection" oder "subtle gaze redirection" bezeichnet). Zur Erzeugung der Aufmerksamkeit erregenden Lichtsignale sind LED-Arraydisplays und Head-Up Displays (HUD) als Projektionsmittel genannt.

Die DE 60009057 T2 offenbart ein Trainingssystem für Anwender, wobei einerseits ein Eyetracking-System die Blickrichtung z.B. eines Kraftfahrzeugfahrers erfassen kann und durch Einblendung eines adaptiven Ereignisses dieser Fahrer erzogen werden kann, seinen Blickwinkel zu verändern und diesen folglich zu einem gewünschten Verhalten zu konditionieren.

In der US 2015 355805 A1 werden verschiedene Ausführungen von optischen Reizen geoffenbart, wobei als Parameter verschiedene Umrissformen, Farben, Blink-Frequenzen und Blinkdauer vorgeschlagen werden, um die Aufmerksamkeit eines Probanden zu erregen.

Die JPH08153284 A beschreibt unter Anderem sinnvolle Frequenzen für visuelle Stimuli zur Anwendung in einer Warnanzeige, deren optischen Blinksignale dazu ausgelegt sind, die Aufmerksamkeit einer Person entsprechend anzuregen.

Die WO 2013 136374 A1 offenbart eine Vorrichtung, mit der die Blickrichtung eines Fahrers durch Anzeige eines Soll-Blickpunktes in einem HUD gelenkt werden kann. Die Position des optischen Signals, z.B. in Form eines Lichtpunkts oder einer Lichtlinie, wird in Abhängigkeit von Fahrzeuggeschwindigkeit und Fahrbahnverlauf im Blickfeld des Fahrers eingespielt.

Die US 2013 0241747 A1 nutzt eine im Armaturenbrett verbaute Projektionseinheit, die in das Fahrer-Gesichtsfeld leuchtende Punkte unterschiedlicher Farbe und/oder Intensität einzublenden vermag, um so die Aufmerksamkeit des Fahrers zu aktivieren und den Blick in eine bestimmte Richtung zu lenken. Als Projektionsfläche wird die Innenfläche der Windschutzscheibe genutzt. Durch Variation der Intensität und der Fahre der Lichtpunkte wird der Blick des Fahrers auf unterschiedliche Bereiche vor dem Fahrzeug gelenkt.

Die DE 10 2010 041961 A1 beschreibt optische Anzeigeeinrichtungen im Fahrzeuginnenraum, mit dem die Aufmerksamkeit des Fahrers auf eine Gefahrensituation gelenkt wird. Dazu wird der Bildschirm des mittig in der Armaturentafel angeordneten Fahrzeug-Informationsdarstellungssystems genutzt, um eine Lichtbewegung/Lichtanimation in Richtung des Fahrersichtfelds, z.B. über sequentielle Aktivierung von Leuchtpunkten zu erzeugen.

Die DE 10 2005 036002 A1 beschreibt ein Verfahren zur Steuerung der Beleuchtungseinrichtung eines Scheinwerfersystems, bei dem die Blickrichtung des Fahrers aufgenommen wird und anschließend die steuerbaren Kraftfahrzeug (Kfz)-Scheinwerfer in diese Blickrichtung ausgerichtet werden.

Die WO 2015 006793 A1 beschreibt ein Fahrerassistenzsystem mit einem in Abhängigkeit der Blickrichtung eines Fahrers eines Fahrzeuges gesteuerten, adaptiven Beleuchtungsmittel.

Es ist eine Aufgabe der vorliegenden Erfindung, ein Verfahren zur Steuerung und/oder zum Trainieren des Fahrverhaltens eines Fahrers beim Führen eines Kraftfahrzeugs bereitzustellen, mit welchem durch eine gezielte Blicklenkung der Fahrer beeinflusst wird, damit dieser situationsgerecht und personalisiert auf die Bedürfnisse der drei visuell beeinflussten kognitiven Grundaufgaben des (Nacht-)Fahrens - nämlich Motorik ansteuern, Situation beurteilen, im Verkehrsraum navigieren - reagieren kann. Insbesondere ist es wünschenswert, mit diesem Verfahren die oben genannten Probleme im Zusammenhang mit der Ausleuchtung durch AFS- bzw. ADB-Scheinwerfer zu verringern bzw. zu beseitigen.

Eine weitere Aufgabe der Erfindung liegt darin, ein Fahrassistenzsystem für ein Kraftfahrzeug zum Ausführen des erfindungsgemäßen Verfahrens bereitzustellen.

Diese Aufgabe wird durch ein Verfahren zur Steuerung und/oder zum Trainieren des Fahrverhaltens eines Fahrers beim Führen eines Kraftfahrzeugs wie eingangs genannt gelöst, welches erfindungsgemäß dadurch gekennzeichnet ist, dass das Hinweissignal dem Fahrer mittels der "Attraktive Flicker"-Methode übermitteln wird, welche die folgenden Schritte umfasst:
- mittels der Scheinwerfermittel oder der mindestens einen Frontleuchte Erzeugen eines Wechsellicht-Orientierungssignals, welches in einer vorgebbaren Position in dem Verkehrsraum vor dem Kraftfahrzeug definiert ist und sich in seiner Amplitude der Lichtintensität für den Fahrer deutlich von einer aktuell vor dem Kraftfahrzeug befindlichen lokalen Umgebungshelligkeit abhebt,
- Aufrechterhalten des Wechsellicht-Orientierungssignals, solange, bis eine Änderung zumindest eines Blickparameters des Fahrers durch die Blickerfassungs-Einrichtung erfasst wird, wodurch ein Anzeichen für die Zuwendung der Aufmerksamkeit des Fahrers auf das Wechsellicht-Orientierungssignal festgestellt wird, und
- Umwandeln des Wechsellicht-Orientierungssignals in ein Wechsellicht-Erhaltungssignal durch Herabsetzen der Frequenz sowie der Amplitude des Wechsellicht-Orientierungssignals auf einen für den auf das Orientierungslichtsignal aufmerksam gewordenen Fahrer erkennbaren Erhaltungswert.

Diese Aufgabe wird weiterhin durch ein Fahrassistenzsystem für ein Kraftfahrzeug zum Ausführen des erfindungsgemäßen Verfahrens gelöst, umfassend (i) mindestens eine den Fahrer betreffende Blickerfassungs-Einrichtung (Eyetrackingeinrichtung), die zum kontinuierlichen Erfassen zumindest eines Blickparameters des Fahrers eingerichtet ist, wobei der Blickparameter aus der Gruppe bestehend aus einer Blickrichtung, einer Blickbewegung und/oder einer Blickdauer ausgewählt ist, (ii) Scheinwerfermittel, die dazu eingerichtet sind, zumindest eine vorgebbare Lichtverteilung vor dem Kraftfahrzeug zu erzeugen, oder mindestens eine Frontleuchte, (iii) Mittel zum Erfassen der aktuellen Verkehrssituation, und (iv) eine Steuerungs- und Auswerteeinrichtung, welche dazu eingerichtet ist, die Scheinwerfermittel oder die mindestens eine Frontleuchte zu steuern, wobei das Fahrassistenzsystem erfindungsgemäß dadurch gekennzeichnet ist, dass die Steuerungs- und Auswerteeinrichtung dazu eingerichtet ist, die folgenden Schritte durchzuführen, wenn die erfassten Blickparameter und/oder die Verkehrssituation im Vergleich zu abgespeicherten diesbezüglichen Normalwerten Abweichungen über vorgebbare Grenzen hinaus aufweisen:
- Übermitteln eines ersten Steuerungssignals an die Scheinwerfermittel oder die mindestens eine Frontleuchte, um ein Wechsellicht-Orientierungssignal, welches in einer vorgebbaren Position vor dem Kraftfahrzeug definiert ist und sich in seiner Amplitude der Lichtintensität für den Fahrer deutlich von einer aktuell vor dem Kraftfahrzeug befindlichen lokalen Umgebungshelligkeit abhebt,
- Aufrechterhalten des Wechsellicht-Orientierungssignals, solange, bis eine Änderung zumindest eines Blickparameters des Fahrers durch die Blickerfassungs-Einrichtung erfasst wird, wodurch ein Anzeichen für die Zuwendung der Aufmerksamkeit des Fahrers auf das Wechsellicht-Orientierungssignal festgestellt wird, und
- Übermitteln eines zweiten Steuerungssignals an die Scheinwerfermittel oder die mindestens eine Frontleuchte, um das Wechsellicht-Orientierungssignals in ein Wechsellicht-Erhaltungssignal durch Herabsetzen der Frequenz sowie der Amplitude des Wechsellicht-Orientierungssignals auf einen für den auf das Orientierungslichtsignal aufmerksam gewordenen Fahrer erkennbaren Erhaltungswert umzuwandeln.

Ein "Attractive Flicker" kann als eine Blinkfolge definiert werden, welche die visuelle Aufmerksamkeit eines Fahrers auf sich lenkt. Die Methode des "Attraktive Flicker" wurde im Detail von Waldner et al. beschrieben (M. Waldner et al., Attractive Flicker - Guiding Attention in Dynamic Narrative Visualizations, 2014, IEEE Transactions on Visualization and Computer Graphics, 20(12): 2456-2465). Bei der zum Zwecke der vorliegenden Erfindung gezielten Blicklenkung zur Beeinflussung des Fahrer-Verhaltens wird auf die, bevorzugt auf für große Displays entwickelte, in narrativem Kontext wirksame Technik des "Attractive Flicker" zurückgegriffen; dies bedeutet, dass der Fahrer nicht notwendigerweise durch die erfindungsgemäß mittels der Scheinwerfermittel oder Frontleuchte erzeugten WechsellichtSignale aus dem narrativem Kontext, also dem "Umgebungsverständnis" oder dem "Situationsbewusstsein", gerissen werden soll.

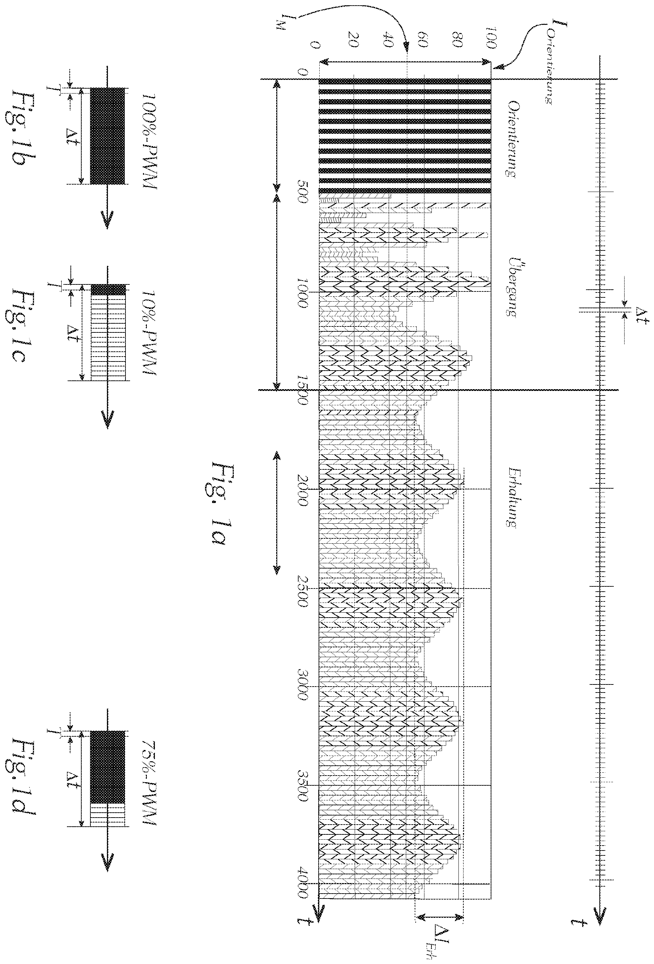

Das Prinzip der "Attractive Flicker"-Methode bei einer Scheinwerferanwendung ist weiter unten in der **Fig. 1** näher erläutert (in Anlehnung an das in Waldner et al., 2015 präsentierte Grundprinzip). Grundsätzlich wird bei dieser mehrstufigen Methodik im Rahmen der vorliegenden Erfindung zur Blicklenkung zuerst ein durch die Scheinwerfermittel erzeugtes sehr intensives und relativ hochfrequentes Wechsellicht-Orientierungssignal ("Orientierung") genutzt, welches sofort, sobald die im Kraftfahrzeug installierte Blickerfassungseinrichtung eine entsprechend gerichtete Änderung einer oder mehrerer der genannten Blickparameter misst, in ein niedriger frequentes, um einen Intensitätsgrundwert pulsierendes, für sich wenig salientes, d.h. im Vergleich zum Wechsellicht-Orientierungssignal wenig auffälliges, aber dennoch dem Bewusstsein zugängliches, Wechsellicht-Erhaltungssignal ("Erhaltung") umgewandelt wird. Die Übergangsphase zwischen "Orientierung" und "Erhaltung" ist in Fig. 1 als "Übergang" bezeichnet.

Ein etabliertes Modell für das Situationsbewusstsein einer Person in einem dynamischen Umfeld ist das von Endsley beschriebene (siehe M. R. Endsley, 1995, Toward a Theorey of Situation Awareness in Dynamic Systems, Human Factors: The Journal of Human Factors and Ergonomics Society, 37, 1, 32-64). Gemäß diesem Modell wird zunächst von einer "Szene" (z.B. die Fahrbahn mit semantischen Attributen und fahrtbezogener Veränderung) in der Umgebung des Fahrzeugs ausgegangen. Diese Szene wird von dem Fahrer des Fahrzeugs erfasst. In dieser "Szene" sind weiterhin zu identifizierende Objekte wie Hindernisse oder andere Verkehrsteilnehmer vorhanden. In einer ersten Stufe ("Objektorientierung"), werden nun die Eigenschaften, der Zustand und Dynamiken relevanter Elemente in der "Szene" erfasst (z.B. eigenes und fremde Fahrzeuge; Hindernisse). In einer darauffolgende zweiten Stufe ("Verstehen"), wird die Szene basierend auf der Verbindung einzelner Elemente der ersten Stufe vom Fahrer verstanden, wodurch Muster gebildet werden. Dies führt zu einer gesamtheitlichen Abbildung der Umgebung einschließlich der Beurteilung der Signifikanz von Objekten und Ereignissen unter Einbeziehung von Erfahrungen und relevanten Zielvorstellungen. In der dritten Stufe ("zukünftiger Zustand") schätzt der Fahrer schließlich auf Grundlage der ersten und zweiten Stufe den zukünftigen Zustand ab (z.B. mögliche Kollisionen) und kann aufgrund dessen eine Entscheidung treffen und entsprechende Handlungen (z.B. Bremsen, Ausweichen, Fahrspur anpassen) setzen.

Das Fahren selbst wird durch verschiedene visuelle und kognitive Gruppen an Aufgaben ("Tasks") beeinflusst. Die Fahrzeug-bezogenen Aufgaben (z.B. Steuern, Gangschalten, Beschleunigen, Bremsen, Blinken) werden durch Szenen-bezogene und die Wahrnehmung des Fahrers betreffende Aufgaben (z.B. Spur halten, Abstand halten, Abstand beurteilen, Geschwindigkeit beurteilen) überlagert. Alle vorgenannten Aufgaben werden wiederum durch Situations-bezogene und das bewusste Verstehen des Fahrers betreffende Parameter (z.B. Berücksichtigung von Verkehrsregeln, Einschätzen des Verkehrsflusses, Anpassung an Wetter- und Lichtverhältnisse, Identifizieren und Bewerten eines Risikos) überlagert.

Die visuelle Aufmerksamkeit des Fahrers, welche dessen Wahrnehmung und somit dessen Situationsbewusstsein prägt, kann einerseits durch die oben angeführten visuellen und kognitiven Gruppen an Aufgaben ("Tasks") und andererseits durch saliente Stimuli gelenkt werden. Bei der vorliegenden Erfindung erfolgt die Lenkung/Steuerung der visuellen Wahrnehmung über den Weg salienter Stimuli zur Blicklenkung, d.h. mittels des erfindungsgemäß eingesetzten "Attraktive Flicker".

Der Einsatz der Methode des "Attraktive Flicker" im Verfahren bzw. im Fahrassistenzsystem gemäß der Erfindung hat enorme Vorteile gegenüber den aus dem Stand der Technik bekannten Verfahren und Systemen. Einerseits wird dank der Erfindung die Dynamik der erzeugten blicklenkenden Lichtsignale (Wechsellicht-Orientierungssignal, Wechsellicht-Erhaltungssignal) nicht als störend empfunden und andererseits wird auch die mögliche Grundintention der allgemeinen Situationsbeurteilung kognitiv, wenn überhaupt, nur minimal beeinträchtigt, d.h. statt auf das erzeugte blicklenkende Lichtsignal ist mit dem kognitiven Kontext der Situationsbeurteilung der Blick auf mögliche Gefahrenmomente lenkbar. Das erfindungsgemäße Verfahren bzw. Fahrassistenzsystem ist besonders bei Nachtfahrten von großem Vorteil und kommt daher in erster Linie dabei zur Anwendung. Weitere vorteilhafte Einsatzsituation sind zum Beispiel Fahrten in Tunnels oder bei Dämmerung.

Grundsätzlich kann also durch das erfindungsgemäße Verfahren bzw. Fahrassistenzsystem die aktuelle Verteilung der kognitiven Beanspruchung des Fahrers gesteuert werden. Das kann zum Beispiel in dem Sinn erfolgen, dass unerfahrene Fahrzeuglenker, die in erster Linie, überproportional und zumeist überschießend, mit der Fahrzeuglenkung beschäftigt sind, frühzeitig auf situationsbeurteilendes Sehen und Erkennen (d.i. die Stärke erfahrener Fahrzeuglenker) hingeführt und somit trainiert werden.

Im Gegenzug können sehr erfahrene Fahrzeuglenker, die überwiegend mit bereits angelernten und verinnerlichten Schemata nach top-down Ansätzen kognitiv arbeiten, für kritische Situationen re-sensibilisiert werden; beispielsweise können bei erfahrenen Fahrern suboptimal angelernte, typischerweise dominante Verhaltensmuster und Situationseinschätzungen ("ich fahre ohne weiteres mit 100 km/h mit Abblendlicht, weil mir dabei noch nie etwas passiert ist") abtrainiert werden oder es kann eine effiziente in-situ Beurteilung bei altersmäßig bedingter Abnahme der sensorischen und kognitiven Leistungsfähigkeit aufrecht erhalten werden.

Unter dem Ausdruck "Normalwert" wie hierin verwendet, sind Werte zu verstehen, die in Abhängigkeit der aktuellen Verkehrssituation als sinnhaft identifiziert wurden.

Blickerfassungseinrichtungen bzw. Eyetrackingeinrichtungen sind aus dem Stand der Technik hinlänglich bekannt und umfassen typischerweise ein Set aus VIS- und IR-Kameras (VIS-Kamera: Umgebung, Kopforientierung; IR-Kamera: Pupillen) sowie ein Steuergerät zur Koordination und Berechnung von Datensätzen bezüglich der Augen- (z.B. Pupillendurchmesser, Akkomodationszustand,) und der Blickparameter (z.B. Fixationen, glatte Verfolgungsvorgänge, Sakkaden, Mikrosakkaden etc. sowie deren Richtung/Orientierung, Amplitude, Dauer, etc.). Als bekannte Hersteller von Remote- und Brillen-basierten Eyetracking-Systemen, die auch für die vorliegende Erfindung geeignet sind, sind beispielsweise Smart Eye (Eyetracker "Aurora"), SensoMotoric Instruments ("EGT", "redn") oder Tobii Tech zu nennen. Das kontinuierliche Erfassen des zumindest einen Blickparameters kann dabei mittels einer im Kraftfahrzeug installierten Blickerfassungs-Einrichtung und/oder mit einer Blickerfassungs-Datenbrille erfolgen. Dementsprechend kann die mindestens eine den Fahrer betreffende Blickerfassungs-Einrichtung als eine im Kraftfahrzeug installierte Blickerfassungs-Einrichtung oder als eine Blickerfassungs-Datenbrille realisiert sein. Zweckmäßigerweise ist die Blickerfassungs-Einrichtung eine im Kraftfahrzeug installierte Blickerfassungs-Einrichtung, da diese vom Fahrer als weniger störend als eine Datenbrille empfunden wird. Durch die entsprechend gestaltete Steuerung und das unmittelbare, permanente Feedback der Blickerfassungseinrichtung ist die Bewertung aktueller Fahrerzustände (z.B. müde, abgelenkt, unaufmerksam) und die Auswertung typischer Fahrblickverhaltensmusters sowie die Bewertung der Trainingseffektivität möglich.

Vorzugsweise wird mittels der zumindest einen Blickerfassungseinrichtung zumindest eine Sakkade des Fahrers erfasst. Unter einer Sakkade wird unter in Fachkreisen eine schnelle und sprunghafte Blickbewegung verstanden.

Der Ausdruck "Wechsellicht-Orientierungssignal, welches sich in seiner Amplitude der Lichtintensität für den Fahrer deutlich von einer aktuell vor dem Kraftfahrzeug befindlichen lokalen Umgebungshelligkeit abhebt" bedeutet, dass die Amplitude der Lichtintensität des Wechsellicht-Orientierungssignals einen Mindestwert eines Helligkeitsunterschieds zur Helligkeit einer vor dem Fahrzeug vorhandenen lokalen Umgebungshelligkeit aufweist, so dass der menschliche Sehapparat das "Wechsellicht-Orientierungssignal" wahrnehmen kann. Die "Lokalität" der Umgebungshelligkeit kann beispielsweise durch die Bildauswertung der Fahrzeugumgebung, z.B. durch eine Lichtintensitätsmessung über ein Kamerasystem, bestimmt werden. Die Helligkeit der vor dem Fahrzeug befindlichen Umgebungshelligkeit wird vornehmlich durch die aktuell durch die Scheinwerfermittel erzeugte Lichtverteilung (z.B. Aufblendlicht mit einem zentralen Helligkeitsmaximum, Abblendlicht und damit verbunden eine Zonentrennung aufgrund der durch den Scheinwerfer erzeugten Hell-Dunkel-Grenze in einen hellen bzw. dunklen Bereich, etc.) sowie die Umgebungslichtverhältnisse (z.B. Straßenbeleuchtung, entgegenkommende Fahrzeuge, blendende Objekte, Umgebungslicht- und Wetterverhältnisse) bestimmt und als Vergleichsgröße zur Verfügung steht.

Der Erhaltungswert des Wechsellicht-Erhaltungssignals kann einem weitgehend konstant empfundenen "Gleichanteil" der aktuell vor dem Kraftfahrzeug befindlichen lokalen Umgebungshelligkeit überlagert sein. Das ist in dem Sinne zu verstehen, dass der wahrgenommene Effektivwert der oszillierenden Signalhelligkeit annähernd dem "Gleichanteil" der Umgebung entspricht oder eine gewollte Helligkeitsdifferenz aufweist.

Gemäß dem Weberschen Gesetz ist die Schwellenwahrnehmung vom Ausgangsreiz, also von der lokalen Umgebungshelligkeit (I₀), abhängig. So liegt der Wert der Weber-Konstante k für die Lichtintensität bei 0,08. Vorzugsweise beträgt der Wert des Helligkeitsunterschieds (ΔI) des Wechsellicht-Orientierungssignals zur aktuell vor dem Kraftfahrzeug befindlichen lokalen Umgebungshelligkeit (I₀) daher zumindest 8 %, bevorzugter liegt der Wert des Helligkeitsunterschieds (ΔI) im Bereich von 8 - 15 %. Die Amplitude der Lichtintensität des Wechsellicht-Orientierungssignals wird daher immer auf den jeweiligen Wert des Helligkeitsunterschieds (ΔI) des Wechsellicht-Orientierungssignals zur jeweils örtlich vorhandenen Umgebungshelligkeit (I₀) angepasst. Entscheidend für die Schwellenwahrnehmung ist der Kontrast, der durch die Beleuchtungsstärke des Wechsellicht-Orientierungssignals entsteht, d.h. je nach Umgebungshelligkeit (z.B. helle Umgebung bei Fernlichtverteilung, dunkle Umgebung im Bereich oberhalb der Licht-Dunkel-Grenze bei Abblendlichtverteilung) wird eine Intensitätszunahme oder Intensitätsabnahme des Wechsellicht-Orientierungssignals gewählt. In den unten angeführten Beispielen sind verschiedene Verkehrsszenen beschrieben, in denen dieser Aspekt näher erläutert ist.

Ergänzend zum oben Gesagten ist noch das Blochsche Gesetz zu erwähnen welches die Helligkeit bzw. die Lichtintensität sowie die Dauer einer visuellen Reizdarbietung mit einer Schwellenwahrnehmung des Beobachters in Zusammenhang bringt, solange die Dauer der Reizdarbietung unter 100 ms liegt. Liegt die Dauer darüber, kommt das Pieron-Gesetz zur Anwendung, wonach die Schwelle für die visuelle Wahrnehmung mit der Quadratwurzel der Zeit ansteigt. Sowohl das Blochsche Gesetz als auch das Pieron-Gesetz besagen, dass ein weniger intensiver Lichtreiz länger dargeboten werden muss, um von einem Beobachter genauso wie ein intensiverer Lichtreiz wahrgenommen zu werden.

Vorzugsweise beträgt der Wert des Helligkeitsunterschieds des Wechsellicht-Erhaltungssignals zur aktuell vor dem Kraftfahrzeug befindlichen lokalen Umgebungshelligkeit zumindest 5%, vorzugsweise 5-10 %.

Gemäß dem erfindungsgemäßen Verfahren kann vorgesehen sein, dass das Wechsellicht-Orientierungssignal eine Frequenz von 10 Hz bis 100 Hz aufweist. In der Praxis hat es sich als günstig erwiesen, wenn das Wechsellicht-Orientierungssignal ein Ein/Aus-Blinksignal mit einer Frequenz von ca. 20 Hz ist.

Gemäß dem erfindungsgemäßen Verfahren kann vorgesehen sein, dass das Wechsellicht-Erhaltungssignal eine Frequenz von 0,5 Hz bis 50 Hz aufweist. In der Praxis hat es sich als günstig erwiesen, dass das Wechsellicht-Erhaltungssignal ein Ein/Aus-Blinksignal mit einer Frequenz von ca. 2 Hz ist.

Um die Zuwendung der Aufmerksamkeit des Fahrers auf das Wechsellicht-Orientierungssignal zu erlangen, ist es von Vorteil, wenn das Wechsellicht-Orientierungssignal mindestens 60 Millisekunden andauert. Das Wechsellicht-Orientierungssignal kann bei bestimmten Varianten des erfindungsgemäßen Verfahrens bis zu 500 Millisekunden andauern.

Bei bestimmten Varianten des erfindungsgemäßen Verfahrens kann es vorgesehen sein, dass das Wechsellicht-Orientierungssignal angepasst wird, wenn innerhalb einer vorgebbaren Zeitdauer keine Änderung des zumindest einen Blickparameters des Fahrers durch die Blickerfassungseinrichtung erfasst wird. Wird also beispielsweise innerhalb einer vorgebbaren Zeitdauer, die zum Beispiel zumindest 60 Millisekunden beträgt, keine Sakkade des Fahrers durch die Eyetrackingeinrichtung gemessen, so wird das Wechsellicht-Orientierungssignal nachträglich angepasst, um eine Zuwendung des Blickes sowie der Aufmerksamkeit des Fahrers zu erreichen. Das Anpassen des Wechsellicht-Orientierungssignals kann insbesondere durch eine Änderung der Amplitude der Lichtintensität, der Frequenz, der Dauer oder des Winkelbereichs des Wechsellicht-Orientierungssignals oder durch eine Kombination davon erfolgen.

Bei einer Weiterbildung des erfindungsgemäßen Verfahrens können dem Fahrer weitere Hinweissignale übermittelt werden, welche aus subtilen optischen Lichtreizen zur Beeinflussung der Blickrichtung des Fahrers (Subtle Gaze Direction (SGD)-Technologie) und/oder Blinklichtsignalen und/oder akustischen Signalen ausgewählt sind. Ein SGD-Hinweissignal kann beispielsweise ein anhaltendes, weniger salientes 10Hz-Signal sein. Einfache Blinklichtsignale können, je nach gewünschter Reaktion, eine Frequenz von z.B. 1, 2 oder 3 Hz aufweisen.

Das Wechsellicht-Orientierungssignal und/oder das Wechsellicht-Erhaltungssignal ist bzw. sind mit Vorteil als animiertes Lichtsymbol realisiert, welche/ s sich gegebenenfalls farblich von der aktuell erzeugten Lichtverteilung unterscheiden kann. Das animierte Lichtsymbol kann beispielsweise ein rotierender Asterisk sein und gegebenenfalls situationsbezogen in ein anders Symbol oder Markierungslicht umspringen. Durch entsprechende Wahl der Lichtblinkfrequenz und der Lichtintensität des animierten Lichtsymbols, die den Helligkeitsunterschied und den Kontrastwechsel zur das Lichtsymbol umgebenden lokalen Umgebungshelligkeit bewirken, kann man das Lichtsymbol die meiste Zeit (besonders bei Kurven ohne Gegenverkehr) nicht im Bewusstsein dominant werden lassen, so dass es nicht als störend empfunden wird, aber dennoch kann durch den "lehrenden" Einsatz der Lichtdynamik der genutzten Blickraum erweitert und eine Blicklenkung bewirkt werden.

Die Position des Wechsellicht-Orientierungssignals und des Wechsellicht-Erhaltungssignals, die wie oben beschrieben zum Beispiel als animierte Lichtsymbole realisiert sein können, ist immer genau dort, wo die visuelle Fahreraufmerksamkeit im Blickfeld des Fahrers sein sollte, jedoch offensichtlich aufgrund des erfassten Blickverhaltens nicht ist. In den unten angeführten Beispielen sind verschiedene Verkehrsszenen im Detail beschrieben, in denen die jeweilige Position der Lichtsignale in Abhängigkeit der Soll-Position der visuellen Fahreraufmerksamkeit beschrieben ist. Die Position der Wechsellichtsignale ist prinzipiell vom aktuellen Grundlichtbild unabhängig. Die einzigen Einschränkungen sind, dass kein anderer Verkehrsteilnehmer geblendet werden darf und dass diese Position durch irgendeinen Teil/Modul der ansteuerbaren Scheinwerfermittel bzw. der ansteuerbaren Frontleuchte(n) entsprechend beleuchtbar ist.

Es kann im Verfahren gemäß der Erfindung vorgesehen sein, dass das Wechsellicht-Orientierungssignal und/oder das Wechsellicht-Erhaltungssignal einen Winkelbereich von 0,1° bis 3°, bevorzugt <1° in vertikaler und in horizontaler Richtung aufweist bzw. aufweisen. Bei einer Weiterbildung kann es vorgesehen sein, dass das Wechsellicht-Orientierungssignal und das Wechsellicht-Erhaltungssignal einen Winkelbereich von 0,1° bis 3°, bevorzugt <1° in vertikaler und in horizontaler Richtung aufweisen. Dementsprechend ist es von Vorteil, wenn die Scheinwerfermittel mindestens einen Scheinwerfer umfassen, wobei die Winkelauflösung zumindest des durch das Signalisieren betroffenen Bereichs des durch den Scheinwerfer ausgeleuchteten Sichtfelds in vertikaler und in horizontaler Richtung in einem Bereich von <<0,01° bis 1°, bevorzugt <0,2° liegt.

Bei einer vorteilhaften Ausgestaltung des erfindungsgemäßen Verfahrens bzw. Fahrassistenzsystems werden das Wechsellicht-Orientierungssignal und das Wechsellicht-Erhaltungssignal mittels mindestens einer in den Scheinwerfermitteln bzw. in der Frontleuchte angeordneten LED-Lichtquelle (kurz als LED bezeichnet) erzeugt. Mit Vorteil werden die "Attractive Flicker"-Signale, umfassend das Wechsellicht-Orientierungssignal und das Wechsellicht-Erhaltungssignal, durch Pulsweitermodulation (PWM) der mindestens einen LED erzeugt. Eine schematische Darstellung eines pulsweitenmodulierten "Attractive Flicker"-Signals ist der Fig. 1a und der Beschreibung hierzu weiter unten zu entnehmen. Dementsprechend weisen die Scheinwerfermittel bzw. die mindestens eine Frontleuchte mit Vorteil zumindest eine pulsweitenmodulierbare LED zum Erzeugen des Wechsellicht-Orientierungssignals und des Wechsellicht-Erhaltungssignals auf. Die Pulsweitenmodulation (PWM) von LEDs, in der Fachwelt auch als PWM-Dimmung bezeichnet, ist den einschlägigen Fachpersonen hinlänglich bekannt. In Scheinwerferanwendungen kommt die PWM-Dimmung in gängiger Weise in Matrix- und Pixellicht-Scheinwerfern zum Einsatz, wobei die Steuerung der LEDs in den Matrix- und Pixellicht-Leuchtmodulen dieser Scheinwerfer über PWM-Signale erfolgt. Grundsätzlich wäre, alternativ zur PWM-Dimmung der LEDs, auch eine Steuerung von LED-Lichtpulsen durch eine direkte analoge Stromänderung möglich; diese alternative Variante hat jedoch im Vergleich zur PWM-Dimmung den Nachteil der Ausbildung störender Farbvariationen aufgrund von Farbort-Verschiebungen.

Wie erwähnt muss die Position des Wechsellicht-Orientierungssignals bzw. des Wechsellicht-Erhaltungssignals durch irgendeinen Teil/Modul der ansteuerbaren Scheinwerfermittel bzw. durch die mindestens eine selbstständige Frontleuchte, die in einem von den Scheinwerfermitteln separaten Gehäuse untergebracht ist, entsprechend beleuchtbar sein. Dies kann beispielsweise durch einen integrierten Teil eines Matrix-, Pixel-, DLP- oder Laser-Scanner-Scheinwerfers erfolgen. Die selbstständige Frontleuchte kann beispielsweise eine unabhängige "Blinklichtzeiger"-Leuchteinheit sein, welche - neben Lösungen mit segmentierten LED Anordnungen - auch aus mechanisch ausrichtbaren Modulen bestehen kann, welche definierte Objekte projizieren können.

Das Wechsellicht-Orientierungssignal und/oder das Wechsellicht-Erhaltungssignal können beispielsweise durch eine im Scheinwerfer integrierte Projektions-Beleuchtungsvorrichtung erzeugt werden, welche entweder neben einer Grundfunktionalität zur Erzeugung von Lichtverteilungen auch bildgebende Eigenschaften aufweist oder diese getrennt von Basisfunktionen ausführt. Zum Beispiel kann die Projektions-Beleuchtungsvorrichtung als scannender Laserstrahl realisiert sein. Eine Laserstrahlprojektion ermöglicht die Erzeugung eines Wechsellicht-Orientierungssignals bzw. Wechsellicht-Erhaltungssignals, welches sich farblich von der Umgebungshelligkeit unterscheidet; beispielsweise könnte ein grünes Wechsellicht-Orientierungs. bzw. Erhaltungssignal auch bei Tage eingesetzt werden. Mittels Laserstrahl erzeugte Attractive Flicker Signale können ferner bei Fahrten im Tunnel oder bei Dämmerung oder bei Gegenlicht von Vorteil sein. Obwohl von einer Projektionsvorrichtung die Rede ist, ist die Erfindung nicht auf Systeme eingeschränkt, die über Abbildungsoptiken das Lichtbild projizieren. Es sind auch Systeme denkbar, die die Straße als Projektionsfläche für die Laserstrahlen nutzen und dort das Lichtbild, z.B. ein Lichtsymbol, "zeichnen", indem der Laserstrahl beispielsweise über einen Mikrospiegel periodisch abgelenkt wird und durch eine entsprechende Modulation der Laserleistung ein für das menschliche Auge flimmerfreies und freiwählbares Bild entsteht. Eine alternative Ausführungsform stellt ein Laser-Projektionssystem für virtuelle Abbildungselemente in Form holographischer Darstellungen dar. Bei einer Ausführungsvariante kann es vorgesehen sein, dass die Projektions-Beleuchtungsvorrichtung eine adaptive Projektions-Vorrichtung ist, mit der das Wechsellicht-Orientierungssignal bzw. das Wechsellicht-Erhaltungssignal erzeugt wird, wobei die Lichtverteilung des projizierten Wechsellicht-Orientierungssignal und/oder das Wechsellicht-Erhaltungssignal adaptiv und situationsbezogen an den Bewegungszustand des Fahrzeugs oder gegebenenfalls auch an den Bewegungszustand von im Blickfeld des Fahrers vorhandener Objekte oder Verkehrsteilnehmer angepasst wird.

Fahrassistenzsysteme sind Zusatzeinrichtungen in Fahrzeugen, die den Fahrer in bestimmten Fahrsituationen unterstützen und entlasten und dabei helfen, kritische Situationen im Vorfeld zu erkennen, insbesondere um das Unfallrisiko zu verringern. Hierzu weisen Fahrassistenzsysteme typischerweise eine Reihe an Subsystemen auf, z.B. eine Rückfahrkamera, ein Parklenkassistenzsystem ("park assist"), ein Totwinkelüberwachungssystem, Umfeldbeobachtungssystem ("front assist"), ein Spurwechselassistenzsystem ("lane assist") etc. Diese Subsysteme nutzen dabei Daten von am oder im Fahrzeug an unterschiedlichen Positionen verbauter Umfeldsensorik, die durch Sensoren, wie Kameras, Radar-Sensoren, Lidar-Sensoren oder dergleichen das Umfeld des Fahrzeuges erfassen und überwachen. Eine typische Funktionalität von Fahrassistenzsystemen besteht darin, dem Fahrer das Umfeld des Fahrzeuges auf der Basis von Daten der Umfeldsensorik mit Hilfe einer von Schnittstellen zur Datenübertragung, z.B. eine Mensch-Maschine-Schnittstelle (*human machine interface* HMI) zu übermitteln. Die HMI-Schnittstelle kann dem Fahrer dann die entsprechenden Informationen z.B. in optischer, haptischer oder akustischer Form übermitteln. Darüber hinaus können noch Daten aus externen mobilen Verbindungen ("Connectivity") wie Positions-/Navigationsdaten (z.B. GPS), Daten betreffend die Kommunikation mit dem Fahrzeug und seiner Umgebung (z.B. car2x, car2car) oder aus weiteren, über Netzwerkanbindung zugänglichen externen Datenbanken beispielhaft zum Verkehrsaufkommen und/oder zum Straßenzustand berücksichtigt werden.

Fahrassistenzsysteme basierend auf einer Assistenz durch Beleuchtung mittels am Kraftfahrzeug frontseitig angeordneten Leuchtmodulen (z.B. Scheinwerfer oder eigenständige Frontleuchten) haben den Vorteil, dass die Assistenz durch Beleuchtung intuitiv ist und, insbesondere in der Nacht oder Dämmerung, das einzige Hilfsmittel ist, welches am und auf den Ort wirkt, der im Fokus der visuellen Wahrnehmung und Aufmerksamkeit des Fahrers liegt, sowie auf jenem Ort, wo dieser Fokus günstigerweise liegen sollte. Die Assistenz durch Beleuchtung bindet den Fahrer daher also viel unmittelbarer als alle anderen Assistenz-Ansätze (z.B. HMI-Schnittstelle in Form eines im Fahrzeuginneren angeordneten Displays) ein.

Das oben Gesagte trifft im Wesentlichen auch für das Fahrassistenzsystem der vorliegenden Erfindung zu, wobei die Informationen erfindungsgemäß in Form von Lichtsignalen nach der Methode des "Attraktive Flicker" mittels der Scheinwerfermittel oder mittels zumindest einer Frontleuchte an den Fahrer weitergegeben werden. Das erfindungsgemäße Fahrassistenzsystem umfasst wie oben definiert Mittel zum Erfassen der aktuellen Verkehrssituation; diese Mittel zum Erfassen der aktuellen Verkehrssituation umfassen zweckmäßigerweise Umfeldsensorikmittel zum Erfassen von Fahrzeugumfelddaten, wobei die Steuerungs- und Auswerteeinrichtung dazu eingerichtet ist, die Fahrzeugumfelddaten zu empfangen, auszuwerten und in der Steuerung der Scheinwerfermittel zu berücksichtigen. Die Umfeldsensorikmittel können dabei jegliche dem Fachmann auf dem Gebiet der Umfeldsensorik bekannten Sensormittel umfassen. Günstigerweise umfassen die Umfeldsensorikmittel dabei zumindest eines der folgenden Sensormittel: optische Sensormittel, Ultraschallsensormittel, Radarsensormittel, LiDAR-Sensormittel, Infrarotsensormittel. Derartige Sensormittel sind dem einschlägigen Fachmann hinlänglich bekannt. Die optischen Sensormittel umfassen vorzugsweise zumindest ein Kamerasystem mit zumindest einer Kamera wie zum Beispiel bildsensorgestützte Front- und Umfeldkameras. Zweckmäßigerweise ist die zumindest eine Kamera des Kamerasystems so angeordnet, dass das Kamerasystem Bilder und/oder Videos im Gesichtsfeld des Fahrers aufnehmen kann. Die Kamera kann die Funktionen einer normalen Kamera, einer Nachtsicht-Kamera und/oder einer Wärmebildkamera, die, basierend auf Nutzung von beispielsweise Langwellen-IR im Bereich von 8-12µm (nach N.Pichon et.al. "All-weather vision for automotive safety: which spectral band?" in VISION 2016 proceedings) zuverlässig Fußgängererkennung bei Nacht und Nebel Bedingungen erlaubt und daher als "Nebelsichtkamera" genutzt werden kann, umfassen, so dass bei allen Lichtverhältnissen und Witterungen eine zuverlässige und qualitativ ausreichende Erfassung der Fahrzeugumgebung und darin befindlicher Objekte bzw. anderer Verkehrsteilnehmer möglich ist.

Ferner kann die zumindest eine Kamera auch dazu eingerichtet sein, Lichtintensitätsmessungen hinsichtlich der Umgebungshelligkeit durchzuführen. Insbesondere kann dadurch die lokale Umgebungshelligkeit, in die das Wechsellicht-Orientierungssignal bzw. Wechsellicht-Erhaltungssignal positioniert werden soll, durch eine Lichtintensitätsmessung über das Kamerasystem, bestimmt werden.

Die im Erfassungsbereich der jeweiligen Umfeldsensorikmittel generierten Umfelddaten werden wie oben erwähnt an die Steuerungs- und Auswerteeinrichtung zur Auswertung weitergeleitet. Die Umfeldsensorikmittel können auch in Kombination miteinander eingesetzt werden, um die verschiedenen Vorteile zu nutzen, z.B. eine Kombination der Daten aus einer Kamera und einem Infrarotsensormittel, und um Menschen und Tiere auf Grundlage ihrer Körperwärme noch besser erfassen zu können.

Bei einer Weiterbildung der Erfindung ist es vorgesehen, dass der Steuerungs- und Auswerteeinheit eine auf einem Expertensystem oder einer künstlichen-Intelligenz-Logik (AI-Logik) basierende Datenbank, vorzugsweise persönliche Charakteristika des Fahrers umfassend, zugeordnet ist, wobei die Steuerungs- und Auswerteeinrichtung dazu eingerichtet ist, Daten aus der Datenbank in der Steuerung der Scheinwerfermittel bzw. der zumindest einen Frontleuchte zu berücksichtigen. Auf diese Weise können der Trainingsstand oder das Trainingsresultat bzw. das persönliche Standardverhalten des jeweiligen Fahrers bei der Steuerung bzw. beim Trainieren des Fahrverhaltens berücksichtigt werden. In der praktischen Anwendung kann das Fahrassistenzsystem unter Berücksichtigung vorhandener Datenbank-Daten auf das spezifische Verhalten des Fahrers (in erster Linie das Blickverhalten und das daraus abgeleitete Fahrverhalten) "lehrend" einwirken und einerseits als selbst lernendes System aus dem Verhalten des Fahrers "lernen". Darüber hinaus können diese Daten aus der Datenbank auch übertragen werden, z.B. über ein Expertensystem mit persönlichen Charakteristiken des Fahrers auf einem Datenträger. Die Steuerung bzw. das Trainieren des Fahrverhaltens sowohl über unmittelbares Feedback zum Fahrerverhalten als auch über eine vorausschauende Einschätzung basierend auf bewerteten Erfahrungsdaten bewirkt im Kombination mit der Methode des "Attractive Flicker" einen besonders effizienten Trainingseffekt im Vergleich zu aus dem Stand der Technik bekannten reinen Salienzeffekten. D.h. ein identifiziertes förderliches Fahrverhalten wird durch wirkungsorientierte "Attractive Flicker"-Lichtsignale unterstützt; die "Attractive Flicker"-Lichtsignale werden aber nicht als störend oder (potentiell) stressig empfunden werden und beeinträchtigen die allgemeine Situationsbeurteilung kognitiv, wenn überhaupt, nur minimal.

Ein beispielhaftes Schema der dem Fahrassistenzsystem unterliegenden Steuerung unter Berücksichtigung der erfassten Umfelddaten, des Blickverhaltens des Fahrers und bestehender Fahrer- und Situationsdaten ist in den beiliegenden Figuren gezeigt und im Detail erläutert.

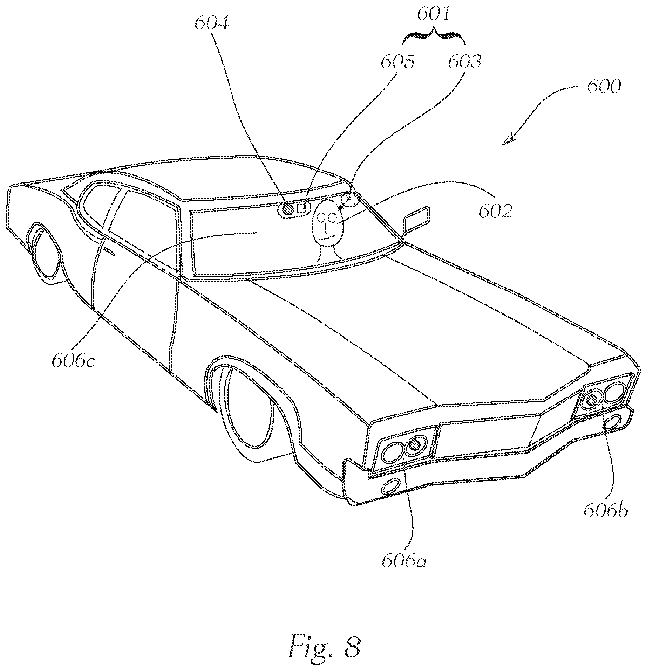

Ein weiterer Gegenstand der Erfindung betrifft ein Kraftfahrzeug, welches ein erfindungsgemäßes Fahrassistenzsystem wie hierin beschrieben umfasst. Das Kraftfahrzeug umfasst zweckmäßigerweise zwei Frontscheinwerfer als Scheinwerfermittel zum Erzeugen des Wechsellicht-Orientierungssignals bzw. des Wechsellicht-Erhaltungssignals. Anstelle der Scheinwerfermittel, kann auch zumindest eine selbstständige Frontleuchte dazu vorgesehen zum Erzeugen des Wechsellicht-Orientierungssignals bzw. des Wechsellicht-Erhaltungssignals vorgesehen sein. Die zumindest eine Frontleuchte kann am Kraftfahrzeug zum Beispiel in unmittelbarer Nähe des Frontscheinwerfers bzw. der Frontscheinwerfer und/oder aber auch in mittiger Position im oberen Bereich der Windschutzscheibe des Kraftfahrzeugs (siehe z.B. Fig. 8) positioniert sein. Das Wechsellicht-Orientierungssignal bzw. das Wechsellicht-Erhaltungssignal können wie oben beschrieben beispielsweise mittels einer in einem oder beiden Frontscheinwerfer/n montierten (adaptiven) Projektions-Beleuchtungsvorrichtung erzeugt werden. Vorzugsweise umfasst jeder der beiden Frontscheinwerfer genau eine adaptive Projektions-Beleuchtungsvorrichtung, da die Konstruktion der Frontscheinwerfer dadurch vereinfacht und raumsparend ausgeführt ist.

Die Erfindung und deren Vorteile werden im Folgenden anhand von nicht einschränkenden Beispielen gemäß der beiliegenden Zeichnung erläutert. Die Zeichnung zeigt in:
Fig. 1a-d eine schematische Darstellung des Grundprinzips der erfindungsgemäß eingesetzten "Attractive Flicker"-Methode am Beispiel einer Pulsweitenmodulation (PWM) einer LED eines Scheinwerfers,
Fig. 2 eine beispielhafte Konfiguration der funktionellen Einheiten zur Steuerung des erfindungsgemäßen Fahrassistenzsystems unter Berücksichtigung der erfassten Umfelddaten, des Blickverhaltens des Fahrers sowie vorgegebener Fahrerdaten,
Fig. 3 eine erste beispielhafte Verkehrsszene, in der das erfindungsgemäße Verfahren veranschaulicht wird,
Fig. 4a-c eine zweite beispielhafte Verkehrsszene, in der das erfindungsgemäße Verfahren veranschaulicht wird,
Fig. 5 eine dritte beispielhafte Verkehrsszene, in der das erfindungsgemäße Verfahren veranschaulicht wird,
Fig. 6 eine vierte beispielhafte Verkehrsszene, in der das erfindungsgemäße Verfahren veranschaulicht wird,
Fig. 7 eine fünfte beispielhafte Verkehrsszene, in der das erfindungsgemäße Verfahren veranschaulicht wird, und
Fig. 8 eine schematische Darstellung eines Kraftfahrzeugs, das mit einem erfindungsgemäßen Fahrassistenzsystem ausgestattet ist.

**Fig. 1a****-d** zeigen eine schematische Darstellung des Prinzips der erfindungsgemäß eingesetzten "Attractive Flicker"-Methode am Beispiel einer Pulsweitenmodulation einer LED-Lichtquelle (kurz LED) eines Scheinwerfers im Zeitverlauf (t). Das in Fig. 1a-d dargestellte Prinzip der "Attractive Flicker"-Methode ist an das in Waldner et al., 2015 präsentierte Grundprinzip angelehnt. Bezugnehmend auf das Diagramm in der Fig. 1a, wird bei dieser mehrstufigen Methodik im Rahmen der vorliegenden Erfindung zur Blicklenkung zuerst ein sehr intensives und relativ hochfrequentes Wechsellicht-Orientierungssignal ("Orientierung") erzeugt, welches sofort, sobald die im Kraftfahrzeug installierte Blickerfassungseinrichtung eine entsprechend gerichtete Änderung einer oder mehrerer Blickparameter des Fahrers misst, in ein niedriger frequentes, um einen Intensitätsgrundwert pulsierendes, für sich wenig salientes, d.h. im Vergleich zum Wechsellicht-Orientierungssignal wenig auffälliges, aber dennoch dem Bewusstsein zugängliches, Wechsellicht-Erhaltungssignal ("Erhaltung") umgewandelt wird. Die Übergangsphase zwischen "Orientierung" und "Erhaltung" ist in Fig. 1a als "Übergang" bezeichnet. Gemäß der Erfindung können das Wechsellicht-Orientierungssignal und das Wechsellicht-Erhaltungssignal mittels Scheinwerfermitteln wie Frontscheinwerfer oder mit Hilfe einer eigenständigen Frontleuchte erzeugt werden. In dem in Fig. 1a gezeigten Beispiel ist die Erzeugung dieser Signale im Zeitverlauf (t) mittels einer pulsweitenmodulierten LED, die im Scheinwerfer bzw. in der Frontleuchte eingebaut ist, dargestellt. Die Ordinate entspricht der vom Menschen (Fahrer) wahrgenommenen Intensität/Helligkeit I_{M}, die durch die Pulsweitenmodulation(PWM)-Dimmung der LED erzielt wird. Die Intensität eines Lichtpunktes bzw. die Lichtverteilung wird im gezeigten Beispiel über die PWM-Dimmung mit einer LED Pulsweite T = 5ms und einer Taktung von 200 Hz gesteuert. Die 5ms-Einzelpulse, die nur als beispielhaft anzusehen sind, werden über eine Zeitdauer integriert und als einziger Helligkeitswert wahrgenommen.

Zunächst wird bei der "Attractive-Flicker"-Methode wie oben erwähnt zur Blicklenkung ein sehr intensives und relativ hochfrequentes Wechsellicht-Orientierungssignal ("Orientierung") mittels der LED erzeugt. Im gezeigten Beispiel wird die LED in 65ms-Zeitabständen (Δt = 65 ms) über einen Zeitraum von 500 ms ein- bzw. ausgeschaltet; dieses Signal wird vom Fahrer als relativ hochfrequentes Ein/ Aus-Blinklichtsignal wahrgenommen. Im gezeigten Beispiel beträgt die Lichtintensität der LED im eingeschalteten/bestromten Zustand 100% vom Intensitätswert I_{Orientierung} (im gezeigten Beispiel bei "100%-PWM") über den Zeitraum Δt = 65 ms; dies ist in Fig. 1b in einer Detailansicht einer 65ms-Zeitperiode dargestellt (100%-PWM; LED-Pulsweite T = 5ms, Taktfrequenz f = 200 Hz; Δt = 65 ms). Der Intensitätswert I_{Orientierung} des Wechsellicht-Orientierungssignals ist in dem in Fig. 1a gezeigten Beispiel mit 100%-PWM ident. In der praktischen Umsetzung orientiert sich der Intensitätswert I_{Orientierung} des Wechsellicht-Orientierungssignals jedoch an der lokalen Umgebungshelligkeit (siehe Beschreibung weiter oben bzw. folgende Beschreibung) und ist nicht automatisch mit 100%-PWM ident.

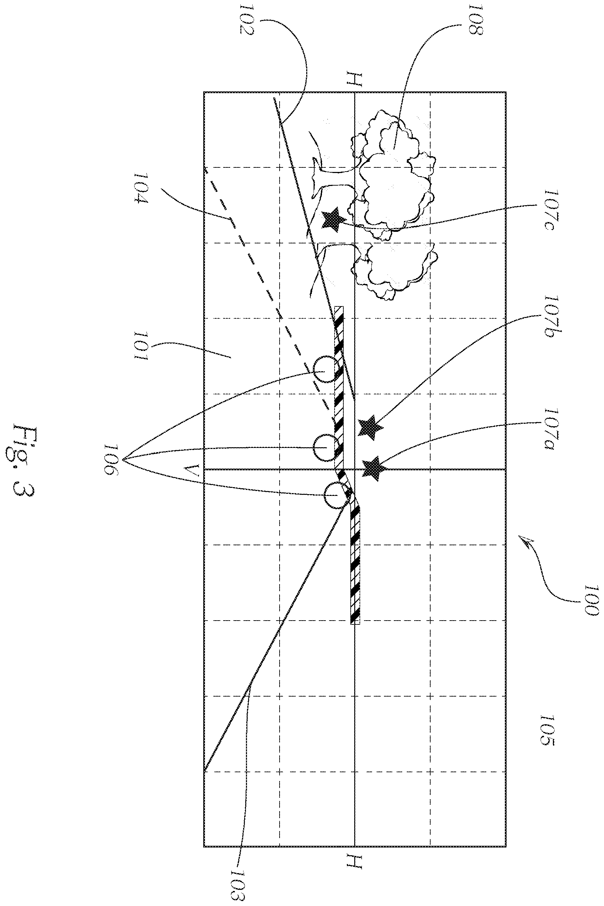

Hinsichtlich der Wahl der Lichtintensität des Wechsellicht-Orientierungssignals wird - mit Verweis auf die obigen Ausführungen - nochmals angemerkt, dass die Amplitude der Lichtintensität (vgl. Intensitätswert I_{Orientierung} in Fig. 1a) des durch die LED erzeugten Wechsellicht-Orientierungssignals einen Mindestwert eines Helligkeitsunterschieds zur Helligkeit einer vor dem Fahrzeug vorhandenen lokalen Umgebungshelligkeit aufweisen muss, so dass der menschliche Sehapparat das Wechsellicht-Orientierungssignal wahrnehmen kann. Die Helligkeit der vor dem Fahrzeug befindlichen Umgebungshelligkeit wird wie oben bereits erwähnt vornehmlich durch die aktuell durch die Scheinwerfermittel erzeugte Lichtverteilung (z.B. Aufblendlicht, Abblendlicht und damit verbunden die Helligkeit im hellen bzw. dunklen Bereich der Hell-Dunkel-Grenze etc.) sowie die Umgebungslichtverhältnisse (z.B. Straßenbeleuchtung, entgegenkommende Fahrzeuge, blendende Objekte, Umgebungslicht- und Wetterverhältnisse) bestimmt. Gemäß dem Weberschen Gesetz ist die Schwellenwahrnehmung vom Ausgangsreiz, also von der lokalen Umgebungshelligkeit (I₀), abhängig. Vorzugsweise beträgt der Wert des Helligkeitsunterschieds (ΔI) des Wechsellicht-Orientierungssignals zur aktuell vor dem Kraftfahrzeug befindlichen lokalen Umgebungshelligkeit (I₀) daher zumindest 8 %, bevorzugter liegt der Wert des Helligkeitsunterschieds (ΔI) im Bereich von 8 - 15 %. Die Amplitude der Lichtintensität des durch die LED erzeugten Wechsellicht-Orientierungssignals wird daher vorzugsweise auf den jeweiligen Wert des Helligkeitsunterschieds (ΔI) des Wechsellicht-Orientierungssignals zur jeweils örtlich vorhandenen Umgebungshelligkeit (I₀) angepasst. Entscheidend für die Schwellenwahrnehmung ist der Kontrast, der durch die Beleuchtungsstärke des Wechsellicht-Orientierungssignals entsteht, d.h. je nach Umgebungshelligkeit (z.B. helle Umgebung bei Fernlichtverteilung, dunkle Umgebung im Bereich oberhalb der Licht-Dunkel-Grenze bei Abblendlichtverteilung) wird eine Intensitätszunahme oder Intensitätsabnahme des Wechsellicht-Orientierungssignals gewählt. In den unten angeführten Beispielen (Fig. 3 - 7) sind verschiedene Verkehrsszenen beschrieben, in denen dieser Aspekt deutlich wird.

Sobald nun das Wechsellicht-Orientierungssignal vom Fahrer wahrgenommen wird, d.h. sobald die im Kraftfahrzeug installierte Blickerfassungseinrichtung eine entsprechend gerichtete Änderung einer oder mehrerer Blickparameter misst, wird das Wechsellicht-Orientierungssignal mittels PWM in ein niedriger frequentes, um einen Intensitätsgrundwert pulsierendes (durch den mit ΔI_{Erh} bezeichneten Pfeil dargestellt), für sich wenig salientes, d.h. im Vergleich zum Wechsellicht-Orientierungssignal wenig auffälliges, aber dennoch dem Bewusstsein zugängliches, Wechsellicht-Erhaltungssignal ("Erhaltung") umgewandelt. Der Blickparameter kann beispielsweise eine Änderung der Blickrichtung, eine Blickbewegung und eine Blickdauer des Fahrers sowie eine Kombination dieser Blickparameter sein (siehe hierzu die Ausführungen weiter oben in der Beschreibung). Die im gezeigten Beispiel ca. 1000 ms andauernde Übergangsphase zwischen "Orientierung" und "Erhaltung" ist in Fig. 1a als "Übergang" bezeichnet.

Fig. 1c zeigt beispielhaft eine 10%-PWM-Dimmung (10% PWM; LED-Pulsweite T = 5ms / f=200Hz; Δt = 65 ms) während der Übergangsphase (siehe Zeitachse bei t = 565 - 640 ms); soll die Intensität der LED bzw. der Lichtverteilung daher 10% vom Intensitätswert I_{Orientierung} über die Periode Δt = 65 ms betragen, wird die LED während dieser Periode in 10% der Zeit betrieben. In Analogie zu Fig. 1c zeigt die Fig. 1d beispielhaft eine 75%-PWM-Dimmung (75% PWM; LED-Pulsweite T = 5ms/f=200Hz; Δt = 65 ms) während der Erhaltungsphase. 65ms-Perioden mit einer 75%-PWM-Dimmung sind in der Fig. 1a in den steigenden und fallenden Flanken des Wechsellicht-Erhaltungssignals zu erkennen; soll also die Intensität der LED bzw. der Lichtverteilung 75% vom Intensitätswert I_{Orientierung} über die Periode Δt = 65 ms betragen, wird die LED während dieser Periode wie in Fig. 1d dargestellt nur in 75% der Zeit betrieben. Es sei noch einmal angemerkt, dass die PWM-Dimmung von LEDs in der Fachwelt allgemein bekannt ist, weshalb eine noch mehr ins Details gehende Erläuterung dieses Prinzips nicht notwendig erscheint.

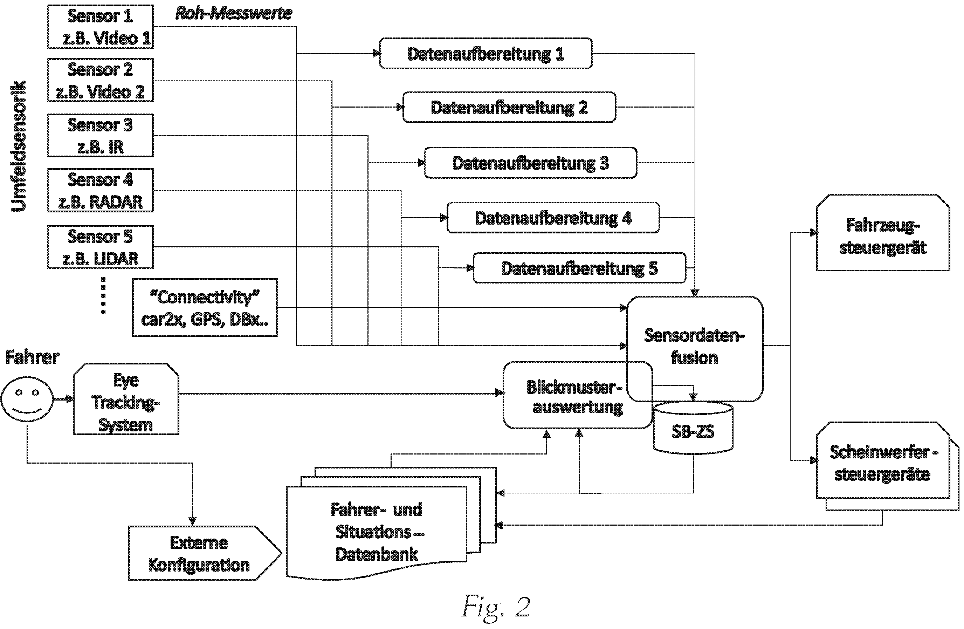

**Fig. 2** zeigt eine beispielhafte Konfiguration der funktionellen Einheiten und Steuerungseinheiten zur Steuerung des erfindungsgemäßen Fahrassistenzsystems unter Berücksichtigung der erfassten Umfelddaten, des Blickverhaltens des Fahrers sowie bestehender Fahrer- und Situationsdaten, in Form eines Flussdiagramms.

Die in Fig. 2 dargestellte Konfiguration umfasst Umfeldsensorikmittel zum Erfassen der aktuellen Fahrzeugumfelddaten, wobei in diesem Beispiel fünf Sensoren 1-5 dargestellt sind. Einem Fachmann wird jedoch klar sein, dass die Umfeldsensorikmittel auch weniger oder mehr Sensoren umfassen können. Die Sensoren 1-5 können beispielsweise optische Sensormittel z.B. in Form von Videokameras (Sensor 1, Sensor 2) sowie IR-, Radar- und LiDar-Sensoren (Sensoren 3-5) sein. Die jeweils durch die Sensoren 1-5 erfassten Fahrzeugumfelddaten werden als Rohdaten oder optional entsprechend aufbereitet (Datenaufbereitung 1, 2, 3, 4 und 5) zusammengeführt und verarbeitet (Sensordatenfusion). In der Sensordatenfusion werden zudem noch Daten aus externen mobilen Verbindungen ("Connectivity") wie Positions-/Navigationsdaten (z.B. GPS), Daten betreffend die Kommunikation mit dem Fahrzeug und seiner Umgebung (z.B. car2x, car2car) oder aus weiteren, über Netzwerkanbindung zugänglichen externen Datenbanken beispielhaft zum Verkehrsaufkommen und/oder zum Straßenzustand berücksichtigt. Die in der Sensordatenfusion gesammelten Datensätze werden einem Fahrzeugsteuergerät sowie den Scheinwerfersteuergeräten zugeführt und in der Steuerung des Fahrzeugs bzw. in der Steuerung der Scheinwerfer berücksichtigt.

Das Fahrassistenzsystem umfasst weiterhin ein Eyetracking-System, das typischerweise ein Set aus VIS- oder IR-Kameras (VIS-Kamera: Umgebung, Kopforientierung; IR-Kamera: Pupillen) sowie ein Steuergerät zur Koordination und Berechnung von Datensätzen bezüglich der Augen- (z.B. Pupillendurchmesser, Akkomodationszustand,) und der Blickparameter (z.B. Fixationen, glatte Verfolgungsvorgänge, Sakkaden, Mikrosakkaden etc. sowie deren Richtung/Orientierung, Amplitude, Dauer, etc.) aufweist. Als bekannte Hersteller von Remote- und Brillen-basierten Eyetracking-Systemen, die auch für die vorliegende Erfindung geeignet sind, sind beispielsweise Smart Eye (Eyetracker "Aurora"), SensoMotoric Instruments ("EGT", "redn") oder Tobii Tech zu nennen. Die mittels dem Eyetracking-System generierten Datensätze werden einer Blickmusterauswertung unterzogen, wobei auch die fusionierten Sensordaten sowie Daten aus einer Fahrer- und Situationsdatenbank in der Blickmusterauswertung berücksichtigt werden.

Die Fahrer- und Situationsdatenbank kann beispielsweise ein Expertensystem oder eine künstliche Intelligenz-Logik (AI-Logik; gemeinsam mit dem Zwischenspeicher und der Blickmusterauswertung die Fähigkeit zu "machine/deep learning"-Methoden nach Stand der Technik) umfassen und zudem persönliche Charakteristika des Fahrers berücksichtigen. Die Fahrer- und Situationsdatenbank kann extern z.B. durch den Fahrer oder eine andere Bedienperson (z.B. Fahrlehrer) konfiguriert werden.

Bei der Blickmusterauswertung erfolgt die Kontextualisierung der durch das Eyetrackingsystem generierten Datensätze unter Berücksichtigung der - unmittelbar gleichzeitig - in interaktiver Abstimmung fusionierten Sensordaten (durch die Überlappung der Felder "Blickmusterauswertung" und "Sensordatenfusion" schematisch dargestellt) sowie der Daten aus der Fahrer- und Situationsdatenbank in Hinblick auf die Bildung eines "maschinellen Situationsbewusstseins". Es erfolgt dabei ein Zugriff auf abgespeicherte Eigenschaften und Verhaltens-, Szenen- und Situationsmuster aus der Fahrer- und Situationsdatenbank sowie auf Datensätze in der Sensordatenfusion betreffend eine aktuell maschinell wahrgenommene Verkehrssituation oder Umfeldszene inklusive Objektallokation. Die Blickmusterauswertung kann beispielsweise in drei Stufen erfolgen: (1) Wahrnehmung/Verstehen einer Situation - (2) Antizipation der Situationsentwicklung -(3) Entscheidung für Maßnahmen. Die Daten aus der Blickmusterauswertung gelangen, abgeglichen und ergänzt mit Ergebnissen der generellen Sensordatenfusion, in einen Zwischenspeicher zur Bildung des Situationsbewusstseins (SB-ZS); das darin gebildete maschinelle Situationsbewusstsein wird unmittelbar ausgelesen und übergibt neu gelernte Situationsmuster wiederum an die Fahrer- und Situationsdatenbank bzw. die Blickmusterauswertung.

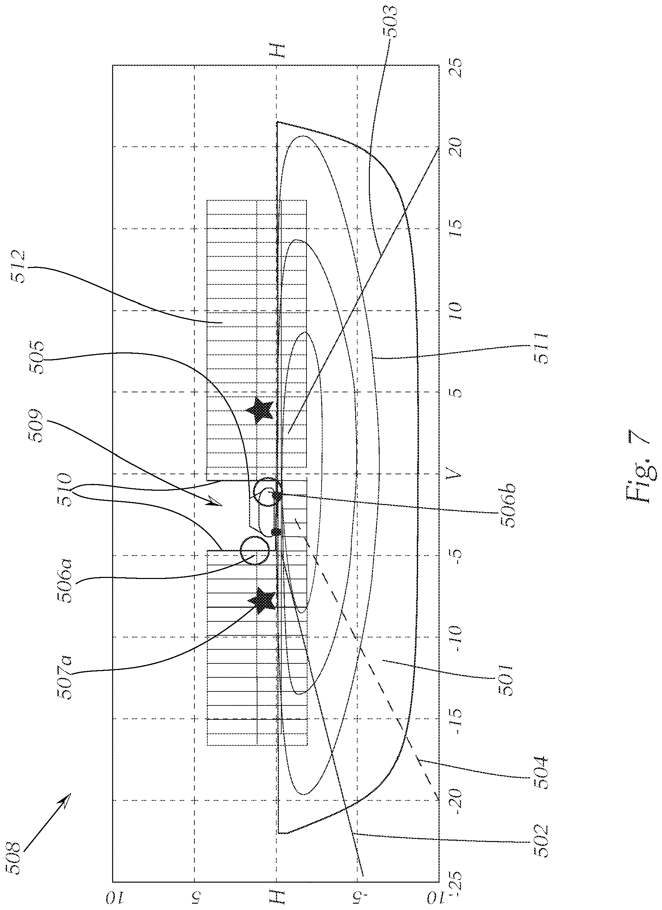

Ausgegeben an die Scheinwerfersteuergeräte werden nicht nur die Ansteuerungswerte einer aktuell gültigen Beleuchtungscharakteristik, sondern auch eine auf Basis aller fusionierten Signale entschiedene, situationsbezogene Vorgehensweise, die entweder in der Fahrer- und Situationsdatenbank oder im Zwischenspeicher zur Bildung des Situationsbewusstseins als Algorithmus hinterlegt ist. Unter dem Begriff "aktuell gültige Beleuchtungscharakteristik" ist eine an die Verkehrssituation angepasste Lichtverteilung zu verstehen; beispielsweise eine Kurvenausleuchtung durch ein Abbiegelicht/Kurvenlichter oder ein blendfreies Fernlicht (siehe z.B. Adaptive Driving Beam (ADB)-Lichtverteilung im Beispiel der Fig. 7). Entsprechend den Steuerungssignalen, die an die Scheinwerfersteuergeräte übermittelt werden, können durch die Scheinwerfer situationsangepasste Attractive Flicker-Lichtsignale (Wechsellicht-Orientierungs- und Erhaltungssignal) wie hierin beschrieben (siehe beispielsweise Fig. 1 und Beschreibung hierzu) erzeugt werden, wodurch eine gezielte Blicklenkung zur Beeinflussung des Fahrerverhaltens in einem dynamischen Umfeld ermöglicht wird.

Mittels der in Fig. 2 beschriebenen Konfiguration können der Trainingsstand oder das Trainingsresultat bzw. das persönliche Standardverhalten des jeweiligen Fahrers bei der Steuerung bzw. beim Trainieren des Fahrverhaltens berücksichtigt werden. Die Daten zum Fahrerverhalten werden zweckmäßigerweise mit einem Zeitstempel gespeichert, so dass eine Bewertung der Trainingsresultate ermöglicht wird. Unter Berücksichtigung vorhandener Daten in der Fahrer- und Situationsdatenbank kann das Fahrassistenzsystem auf das spezifische Verhalten des Fahrers (in erster Linie das Blickverhalten und das daraus abgeleitete Fahrverhalten) "lehrend einwirken und einerseits als selbst lernendes System aus dem Verhalten des Fahrers "lernen". Durch die entsprechend gestaltete Steuerung und das unmittelbare, permanente Feedback über das Eyetrackingsystem bzw. die Blickmusterauswertung ist die Bewertung aktueller Fahrerzustände (z.B. müde, abgelenkt, unaufmerksam) und die Auswertung typischer Fahrblickverhaltensmusters sowie die Bewertung der Trainingseffektivität möglich. Die Steuerung bzw. das Trainieren des Fahrverhaltens sowohl über unmittelbares Feedback zum Fahrerverhalten als auch über eine vorausschauende Einschätzung basierend auf bewerteten Erfahrungsdaten bei der Blickmusterauswertung bewirkt im Kombination mit der Methode des "Attractive Flicker" einen besonders effizienten Trainingseffekt im Vergleich zu aus dem Stand der Technik bekannten reinen Salienzeffekten. D.h. ein identifiziertes förderliches Fahrverhalten wird durch wirkungsorientierte "Attractive Flicker"-Lichtsignale unterstützt; die "Attractive Flicker"-Lichtsignale werden wie erwähnt aber nicht als störend oder (potentiell) stressig empfunden werden und beeinträchtigen die allgemeine Situationsbeurteilung durch den Fahrer selbst kognitiv, wenn überhaupt, nur minimal.

In den **Fig. 3 bis 7** sind verschiedene Verkehrsszenen dargestellt, die im Folgenden im Detail beschrieben werden und in denen die jeweilige Position der "Attractive Flicker"-Lichtsignale (Wechsellicht-Orientierungssignal, Wechsellicht-Erhaltungssignal) in Abhängigkeit der Soll-Position der visuellen Fahreraufmerksamkeit näher erläutert ist. Die in der Offenbarung und auch in der Beschreibung zu den Fig. 3-7 verwendeten Begriffe "Attractive Flicker-Lichtsignal" oder "Attractive Flicker-Signal" beziehen sich auf die mittels der "Attractive Flicker"-Methode erzeugten und weiter oben bereits ausführlich beschriebenen blicklenkenden Lichtsignale, umfassend ein Wechsellicht-Orientierungssignal und Wechsellicht-Erhaltungssignal; auf die detaillierte Beschreibung hierzu in dieser Offenbarung wird verwiesen. Die Position des Wechsellicht-Orientierungssignals und des Wechsellicht-Erhaltungssignals, ist immer genau dort, wo die visuelle Fahreraufmerksamkeit im Blickfeld des Fahrers sein sollte, jedoch, wie aufgrund des erfassten Blickverhaltens offensichtlich erkennbar, nicht ist. In den Fig. 3. bis 7 ist die IstPosition der visuellen Fahreraufmerksamkeit (d.h. die tatsächliche Blickposition) schematisch in Form eines Kreises dargestellt. Die Soll-Position der visuellen Fahreraufmerksamkeit (d.h. die gewünschte Blickposition) und damit die Position des Wechsellicht-Orientierungssignals und des Wechsellicht-Erhaltungssignals (Attractive Flicker-Lichtsignale) ist hingegen schematisch als schwarzer Stern dargestellt. Das Wechsellicht-Orientierungssignal und das Wechsellicht-Erhaltungssignal können in der praktischen Umsetzung beispielsweise als animiertes Lichtsymbol realisiert sein, das sich gegebenenfalls farblich von der aktuell erzeugten Lichtverteilung unterscheiden oder situationsbezogen in ein anderes Symbol oder Markierungslicht umspringen kann.

Die Position der Attractive-Flicker-Lichtsignale ist prinzipiell vom aktuellen Grundlichtbild unabhängig. Die einzigen Einschränkungen sind, dass kein anderer Verkehrsteilnehmer geblendet werden darf und dass diese Position durch irgendeinen Teil/Modul der ansteuerbaren Scheinwerfermittel bzw. der ansteuerbaren Frontleuchte(n) entsprechend beleuchtbar ist. Durch entsprechende Wahl der Lichtblinkfrequenz und der Lichtintensität der Attractive Flicker-Signale, insbesondere des Wechsellicht-Orientierungssignals, die den Helligkeitsunterschied und den Kontrastwechsel zur umgebenden lokalen Umgebungshelligkeit bewirken, kann man das Wechsellicht-Orientierungssignal die meiste Zeit (besonders bei Kurven ohne Gegenverkehr) nicht im Bewusstsein dominant werden lassen, so dass es nicht als störend empfunden wird, aber dennoch kann durch den "lehrenden" Einsatz der Lichtdynamik der genutzten Blickraum erweitert und eine Blicklenkung bewirkt werden.

**Fig. 3** zeigt eine erste beispielhafte Verkehrsszene 100 einer Nachtfahrt aus der Perspektive des Fahrers mit einer Zentralprojektion einer Straße 101 mit linkem Straßenrand 102 und rechtem Straßenrand 103 sowie einem Mittelstreifen 104. Ebenfalls dargestellt ist die Hell-Dunkel-Grenze 105 des Abblendlichts. Aufgrund der kontinuierlichen Lageänderung von Hell-Dunkel-Grenzen, liegt die tatsächliche Blickposition 106 des Fahrers häufig in diesem "unruhigen" Bereich der Hell-Dunkel-Grenze. Mittels Attractive-Flicker-Lichtsignale 107a-c gemäß der Erfindung kann die Blickposition und damit die visuelle Aufmerksamkeit des Fahrers auf relevante Bereiche in der Verkehrsszene gelenkt werden. Beispielsweise können die Attractive-Flicker-Lichtsignale 107a, 107b im Bereich oberhalb der Licht-Dunkel-Grenze positioniert werden, so dass entgegenkommende Fahrzeuge rasch vom Fahrer wahrgenommen werden können. Ferner kann die Blickrichtung des Fahrers mittels dem Attractive-Flicker-Lichtsignal 107c auf die Baumgruppe 108 am linken Straßenrand 102 gelenkt werden, um den Fahrer auf diese Baumgruppe 108 und möglichen Wildwechsel aufmerksam zu machen.

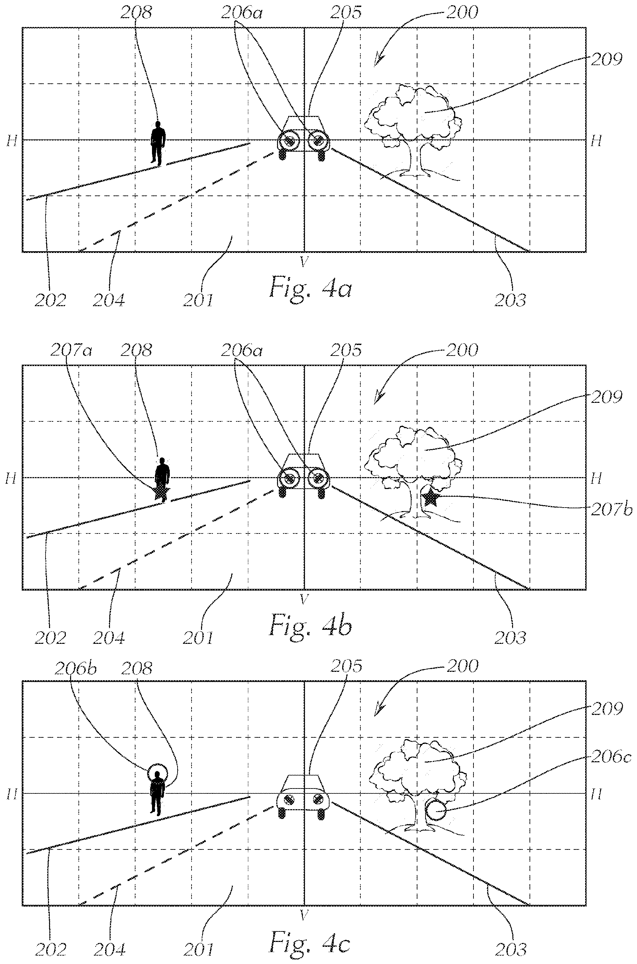

**Fig. 4a****-c** zeigen eine zweite beispielhafte Verkehrsszene 200 einer Nachtfahrt aus der Perspektive des Fahrers mit einer Zentralprojektion einer Straße 201 mit linkem Straßenrand 202 und rechtem Straßenrand 203 sowie einem Mittelstreifen 204. Die tatsächliche Blickposition 206a des Fahrers und somit dessen visuelle Aufmerksamkeit ist im standardmäßigen Fahrverhalten zumeist auf die Heckleuchten des vorausfahrenden Fahrzeugs 205 gerichtet (siehe Fig. 4a). Um den Fahrer nun auf relevante Objekte oder mögliche Hindernisse in der Fahrzeugumgebung bzw. im eigenen Fahrbereich, wie eine auf dem linken Straßenrand 202 entgegenkommende Person 208 oder einen auf dem rechten Fahrbahnrand 203 befindlichen Baum 209 aufmerksam zu machen, kann die Blickposition des Fahrers und somit dessen visuelle Aufmerksamkeit mittels der Attractive-Flicker-Signale 207a bzw. 207b (siehe Fig. 4b) auf die Person 208 bzw. den Baum 209 gelenkt werden (siehe gewünschte und mittels Attractive-Flicker-Lichtsignal 207a bzw. 207b gelenkte Fahrer-Blickposition 206b bzw. 206c in Fig. 4c).

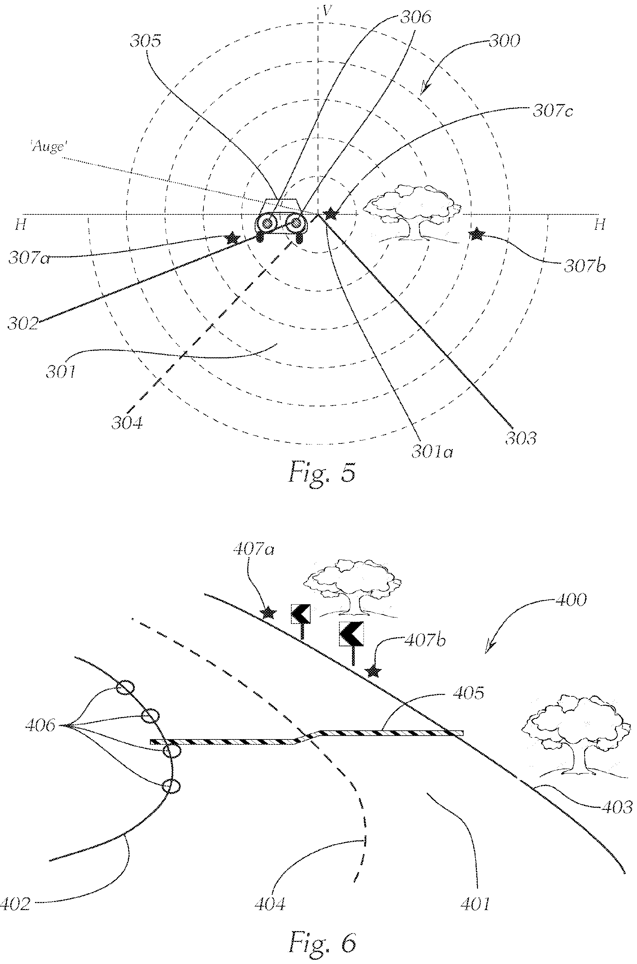

**Fig. 5** zeigt eine dritte beispielhafte Verkehrsszene 300 einer Nachtfahrt aus der Perspektive des Fahrers mit einer Zentralprojektion einer Straße 301 mit linkem Straßenrand 302 und rechtem Straßenrand 303 sowie einem Mittelstreifen 304 und einem entgegenkommenden Fahrzeug 305. In Fig. 5 ist ferner die Augen-Position ('Auge') des entgegenkommenden Fahrzeuglenkers des Fahrzeugs 305 schematisch dargestellt, d.h. in Abhängigkeit der jeweiligen Positionen und Abstände der beiden aufeinander zufahrenden Fahrzeuge. Die gestrichelten Kreise verdeutlichen die Abstände zwischen den beiden Fahrzeugen, d.h. je geringer der Abstand ist, umso geringer ist der Kreisradius. In der Fig. 5 sind zwei Situationen (i) und (ii) veranschaulicht. Der Blick des Fahrers ist nach standardgemäßen Fahrverhalten normalerweise in beiden Situationen direkt auf die Fahrzeugscheinwerfer des entgegenkommenden Fahrzeugs 305 gerichtet (siehe Ist-Blickposition 306), wodurch unnötigerweise auch der Blick lange im Bereich des Lichtkegels des Fahrzeugs 305 liegt und daher sehr rasch eine Adaption des Auges an eine höhere Helligkeit als für den eigenen Fahrbahnbereich sinnvoll ist erfolgt. Dies hat den Nachteil, dass die Erkennbarkeit von Objekten im eigenen Fahrbereich verringert wird. Der Blick des Fahrers soll nun weg von der Lichtquelle, also weg von den Scheinwerfern, gelenkt werden. In Situation (i) ist das entgegenkommende Fahrzeug 305 noch weit weg; in dieser Situation wird die Blickposition des Fahrers und somit dessen visuelle Aufmerksamkeit mittels der Attractive-Flicker-Signale 307a bzw. 307b auf eine Blickposition außerhalb des linken bzw. rechten Fahrbahnrands 302, 303 gelenkt. Auf diese Weise kann der Fahrer dazu trainiert werden, nicht nur auf entgegenkommende Fahrzeuge zu achten, sondern auch die Umgebung besser wahrzunehmen. Das entgegenkommende Fahrzeug 305 wird dabei nicht durch die Attractive-Flicker-Signale 307a, 307b geblendet. Kommt das Fahrzeug 305 nun näher und verringert sich der Abstand zwischen den beiden entgegenkommenden Fahrzeugen (Situation (ii)), tendieren Fahrer normalerweise dazu, direkt in die Scheinwerferlichtquelle des Fahrzeugs 305 zu blicken und riskieren dabei wiederum geblendet zu werden; in diesem Fall wird ein Attractive-Flicker-Signal 307c so positioniert, dass die Blicklenkung des Fahrers weg von den Scheinwerfern des Fahrzeugs 305 auf den rechten Fahrbahnrand 301a in die Ferne gerichtet wird. Das entgegenkommende Fahrzeug 305 wird dabei durch das Attractive-Flicker-Signal 307c, nicht geblendet.

**Fig. 6** zeigt eine vierte beispielhafte Verkehrsszene 400 einer Kurvenfahrt bei Nacht aus der Perspektive des Fahrers mit einer Straße 401 mit linkem Straßenrand 402 und rechtem Straßenrand 403 sowie einem Mittelstreifen 404. Die Straße 401 macht eine Linkskurve. Ebenfalls dargestellt ist die Hell-Dunkel-Grenze 405 des Abblendlichts. Bei Linkskurven orientieren sich Fahrer standardmäßig an der Mittellinie oder am linken Fahrbahnrand; dies ist durch die Ist-Blickpositionen 406 dargestellt. Wenn in so einem Fall ein Fahrzeug entgegenkommt, liegt die Aufmerksam bei diesem und unnötigerweise auch der Blick lange im Bereich des Lichtkegels des Fahrzeugs, wodurch sehr rasch eine Adaption des Auges an eine höhere Helligkeit als für den eigenen Fahrbahnbereich sinnvoll ist erfolgt. Mittels Attractive-Flicker-Lichtsignalen 407a, 407b gemäß der Erfindung kann die Blickposition und damit die visuelle Aufmerksamkeit des Fahrers bei Linkskurven auf den rechten Straßenrand 403 in der Verkehrsszene gelenkt werden. Im gezeigten Beispiel sind die Attractive-Flicker-Lichtsignale 407a, 407b im Bereich oberhalb der Licht-Dunkel-Grenze 405 entlang des rechten Straßenrands 403 positioniert, so dass der Fahrer auf Objekte im oder nebst dem eigenen Fahrbahnbereich aufmerksam wird. Dieses Beispiel illustriert sehr gut, wie das Standardverhalten eines Fahrers mittels Attractive-Flicker-Signalen beeinflusst werden kann, ohne den Fahrer jedoch aus dem "Umgebungsverständnis" bzw. "Situationsbewusstsein" zu reißen oder abzulenken, was insbesondere bei Kurvenfahrten von Bedeutung ist.

**Fig. 7** zeigt eine fünfte beispielhafte Verkehrsszene 500 einer Nachtfahrt aus der Perspektive des Fahrers mit einer Zentralprojektion einer Straße 501 mit linkem Straßenrand 502 und rechtem Straßenrand 503 sowie einem Mittelstreifen 504 und einem entgegenkommenden Fahrzeug 505. Die in Fig. 7 beschriebene Verkehrsszene illustriert die Blicklenkung des Fahrers mittels Attractive Flicker-Lichtsignalen, wenn der Fahrer ein Fahrzeug mit einem LED-Matrix-ADB-Scheinwerfer mit eingeschaltetem Fernlicht (ADB-Fernlichtverteilung 508) lenkt. Die ADB-Fernlichtverteilung 508 als Überlagerung einer Vorfeldausleuchtung 511 und den Matrix-Leuchtelementen 512 des ADB-Scheinwerfers sind aus der Fig. 7 ersichtlich. Um den Fahrer des entgegenkommenden Fahrzeugs 505 nicht mit dem Fernlicht zu blenden, ist jener Bereich des Verkehrsraums nach an sich bekannter Weise automatisch aus der Fernlichtverteilung ausgeblendet, die den Fahrer des entgegenkommenden Fahrzeugs 505 stören würde. Die durch den Ausblendbereich 509 gebildete vertikale Hell-Dunkel-Grenze 510 ist ebenfalls schematisch dargestellt. Im Standardverhalten tendieren Fahrer dazu, den Blick auf die vertikale Hell-Dunkel-Grenze 510 dem bestimmenden Kontrastunterschied bzw. direkt auf die Scheinwerfer des entgegenkommenden Fahrzeugs 505 zu richten (siehe Ist-Blickpositionen 506a bzw. 506b), wodurch die Erkennbarkeit von Objekten im eigenen Fahrbereich bzw. in dem durch die Fernlichtverteilung 508 ausgeleuchteten Bereich verringert wird. Mittels Attractive-Flicker-Lichtsignalen 507a bzw. 507b gemäß der Erfindung kann die Blickposition und damit die visuelle Aufmerksamkeit des Fahrers weg von der vertikalen Hell-Dunkel-Grenze 505 bzw. weg von dem entgegenkommenden Fahrzeug 505 an den linken bzw. rechten Straßenrand 502 bzw. 503 gelenkt werden. Auch dieses Beispiel illustriert sehr gut, wie das Standardverhalten eines Fahrers mittels Attractive-Flicker-Signalen beeinflusst werden kann, ohne den Fahrer jedoch aus dem "Umgebungsverständnis" bzw. "Situationsbewusstsein" zu reißen oder abzulenken.

**Fig. 8** zeigt eine vereinfachte schematische Darstellung eines Kraftfahrzeugs 600 das mit einem erfindungsgemäßen Fahrassistenzsystem 601 zum Ausführen des Verfahrens basierend auf der Attractive-Flicker-Methode gemäß der Erfindung ausgestattet ist. Das Kraftfahrzeug 600 bzw. das Fahrassistenzsystem 601 umfasst eine den Fahrer 602 betreffende Blickerfassungs-Einrichtung 603 (Eyetracking-System 603), mit dem zumindest ein Blickparameter des Fahrers kontinuierlich erfasst wird. Hinsichtlich der Details zu Eyetracking-Systemen und den damit erfassbaren Blickparametern wird auf die obigen Ausführungen verwiesen. Das Fahrassistenzsystem 601 umfasst ferner zumindest eine mittig im oberen Bereich der Windschutzscheibe 606c eingebaute eigenständige Frontleuchte 604 sowie eine ebenfalls mittig im oberen Bereich der Windschutzscheibe 606c eingebaute Frontkamera 605 als Mittel zum Erfassen der aktuellen Verkehrssituation. Das Fahrassistenzsystem 601 erfasst ferner eine nicht näher dargestellte Steuerungs- und Auswerteeinrichtung, welche dazu eingerichtet ist, die Frontleuchte 604 zu steuern. Diese Steuerungs- und Auswerteeinrichtung ist dazu eingerichtet ist, die folgenden Schritte auf Grundlage der hierin beschriebenen Attractive-Flicker-Methode durchzuführen, wenn die erfassten Blickparameter und/oder die Verkehrssituation im Vergleich zu abgespeicherten diesbezüglichen Normalwerten Abweichungen über vorgebbare Grenzen hinaus aufweisen, nämlich:
- Übermitteln eines ersten Steuerungssignals an die Frontleuchte 604, um ein Wechsellicht-Orientierungssignal, welches in einer vorgebbaren Position vor dem Kraftfahrzeug 600 definiert ist und sich in seiner Amplitude der Lichtintensität für den Fahrer deutlich von einer aktuell vor dem Kraftfahrzeug befindlichen lokalen Umgebungshelligkeit abhebt,
- Aufrechterhalten des Wechsellicht-Orientierungssignals, solange, bis eine Änderung zumindest eines Blickparameters des Fahrers 602 durch das Eyetracking-System 603 erfasst wird, wodurch die Zuwendung der Aufmerksamkeit des Fahrers auf das Wechsellicht-Orientierungssignal festgestellt wird, und
- Übermitteln eines zweiten Steuerungssignals an die Frontleuchte 604, um das Wechsellicht-Orientierungssignals in ein Wechsellicht-Erhaltungssignal durch Herabsetzen der Amplitude des Wechsellicht-Orientierungssignals auf einen für den auf das Orientierungslichtsignal aufmerksam gewordenen Fahrer 602 erkennbaren Erhaltungswert umzuwandeln.

Einem Fachmann wird klar sein, dass anstelle der Frontleuchte 604 die Lichtfunktionen der Frontscheinwerfer 606a, 606b des Kraftfahrzeugs 606 zum gesteuerten Erzeugen des Wechsellicht-Orientierungssignals und des Wechsellicht-Erhaltungssignals auf Grundlage der Attractive-Flicker-Methode eingesetzt werden können. Außerdem ist es möglich, die Frontleuchte 604 an einer anderen günstigen Position als oberhalb der Windschutzscheibe 606c im Fahrzeug zu positionieren, zum Beispiel unterhalb der Frontscheinwerfer 606a, 606b. Einem Fachmann wird ferner klar sein, dass das Kraftfahrzeug mit der in Fig. 2 beschriebenen beispielhaften Konfiguration der funktionellen Einheiten zur Steuerung des erfindungsgemäßen Fahrassistenzsystems ausgestattet sein kann.

Die oben genannten Beispiele sind nur wenige unter vielen und daher nicht als einschränkend anzusehen.

Die Bezugszeichen in den Ansprüchen dienen lediglich zum besseren Verständnis der vorliegenden Erfindung und bedeuten auf keinen Fall eine Einschränkung der vorliegenden Erfindung.

## Patentansprüche

1. Verfahren zur Steuerung und/oder zum Trainieren des Fahrverhaltens eines Fahrers beim Führen eines Kraftfahrzeugs, wobei das Kraftfahrzeug aufweist: (i) mindestens eine den Fahrer betreffende Blickerfassungseinrichtung, (ii) Scheinwerfermittel, die dazu eingerichtet sind, mindestens eine vorgebbare Lichtverteilung vor dem Kraftfahrzeug zu erzeugen oder mindestens eine Frontleuchte, und (iii) Mittel zum Erfassen der aktuellen Verkehrssituation, wobei das Verfahren umfasst:
kontinuierliches Erfassen zumindest eines Blickparameters des Fahrers mittels der mindestens einen Blickerfassungs-Einrichtung (Eyetrackingeinrichtung), wobei der Blickparameter aus der Gruppe bestehend aus einer Blickrichtung, einer Blickbewegung und einer Blickdauer des Fahrers sowie einer Kombination dieser Blickparameter ausgewählt ist,
und zumindest ein Hinweissignal für den Fahrer ausgelöst wird, falls die erfassten Blickparameter und/oder die Verkehrssituation im Vergleich zu abgespeicherten diesbezüglichen Normalwerten Abweichungen über vorgebbare Grenzen hinaus aufweisen,
wobei das Verfahren **dadurch gekennzeichnet ist, dass**
das Hinweissignal dem Fahrer mittels der "Attraktive Flicker"-Methode übermitteln wird, welche die folgenden Schritte umfasst:
mittels der Scheinwerfermittel oder der mindestens einen Frontleuchte Erzeugen eines Wechsellicht-Orientierungssignals, welches in einer vorgebbaren Position in dem Verkehrsraum vor dem Kraftfahrzeug definiert ist und sich in seiner Amplitude der Lichtintensität für den Fahrer deutlich von einer aktuell vor dem Kraftfahrzeug befindlichen lokalen Umgebungshelligkeit abhebt,
Aufrechterhalten des Wechsellicht-Orientierungssignals, solange, bis eine Änderung zumindest eines Blickparameters des Fahrers durch die Blickerfassungs-Einrichtung erfasst wird, wodurch ein Anzeichen für die die Zuwendung der Aufmerksamkeit des Fahrers auf das Wechsellicht-Orientierungssignal festgestellt wird, und
Umwandeln des Wechsellicht-Orientierungssignals in ein Wechsellicht-Erhaltungssignal durch Herabsetzen der Frequenz sowie der Amplitude des Wechsellicht-Orientierungssignals auf einen für den auf das Orientierungslichtsignal aufmerksam gewordenen Fahrer erkennbaren Erhaltungswert.

2. Verfahren nach Anspruch 1, **dadurch gekennzeichnet, dass** mittels der zumindest einen Blickerfassungseinrichtung zumindest eine Sakkade erfasst wird.

3. Verfahren nach Anspruch 1 oder 2, **dadurch gekennzeichnet, dass** der Erhaltungswert des Wechsellicht-Erhaltungssignals einem Gleichanteil der aktuell vor dem Kraftfahrzeug befindlichen lokalen Umgebungshelligkeit überlagert ist.

4. Verfahren nach einem der Ansprüche 1 bis 3, **dadurch gekennzeichnet, dass** der Wert des Helligkeitsunterschieds des Wechsellicht-Orientierungssignals zur aktuell vor dem Kraftfahrzeug befindlichen lokalen Umgebungshelligkeit zumindest 8 %, vorzugsweise zwischen 8 % und 15%, beträgt.

5. Verfahren nach einem der Ansprüche 1 bis 4, **dadurch gekennzeichnet, dass** der Wert des Helligkeitsunterschieds des Wechsellicht-Erhaltungssignals zur aktuell vor dem Kraftfahrzeug befindlichen lokalen Umgebungshelligkeit zumindest 5 %, bevorzugt 5-10 % beträgt.

6. Verfahren nach einem der Ansprüche 1 bis 5, **dadurch gekennzeichnet, dass** das Wechsellicht-Orientierungssignal eine Frequenz von 10 Hz bis 100 Hz aufweist.

7. Verfahren nach einem der Ansprüche 1 bis 6, **dadurch gekennzeichnet, dass** das Wechsellicht-Orientierungssignal ein Ein/Aus-Blinksignal mit einer Frequenz von ca. 20 Hz ist.

8. Verfahren nach einem der Ansprüche 1 bis 4, **dadurch gekennzeichnet, dass** das Wechsellicht-Erhaltungssignal eine Frequenz von 0,5 Hz bis 50 Hz aufweist.

9. Verfahren nach einem der Ansprüche 1 bis 8, **dadurch gekennzeichnet, dass** das Wechsellicht-Erhaltungssignal ein Ein/Aus-Blinksignal mit einer Frequenz von ca. 2 Hz ist.

10. Verfahren nach einem der Ansprüche 1 bis 7, **dadurch gekennzeichnet, dass** das Wechsellicht-Orientierungssignal mindestens 60 Millisekunden und vorzugsweise bis zu 500 Millisekunden andauert.

11. Verfahren nach einem der Ansprüche 1 bis 10, **dadurch gekennzeichnet, dass** das Wechsellicht-Orientierungssignal angepasst wird, wenn innerhalb einer vorgebbaren Zeitdauer keine Änderung des zumindest einen Blickparameters des Fahrers durch die Blickerfassungseinrichtung erfasst wird und das Anpassen des Wechsellicht-Orientierungssignals durch eine Änderung der Amplitude der Lichtintensität, der Frequenz, der Dauer oder des Winkelbereichs des Wechsellicht-Orientierungssignals oder eine Kombination davon erfolgt.

12. Verfahren nach einem der Ansprüche 1 bis 11, **dadurch gekennzeichnet, dass** dem Fahrer weitere Hinweissignale übermittelt werden, welche aus subtilen optischen Lichtreizen zur Beeinflussung der Blickrichtung des Fahrers (Subtle Gaze Direction (SGD)-Technologie) und/oder Blinklichtsignalen und/oder akustischen Signalen ausgewählt sind.

13. Verfahren nach einem der Ansprüche 1 bis 12, **dadurch gekennzeichnet, dass** das Wechsellicht-Orientierungssignal und/ oder das Wechsellicht-Erhaltungssignal als animiertes Lichtsymbol realisiert ist bzw. sind, welche/s sich gegebenenfalls farblich von der aktuell erzeugten Lichtverteilung unterscheidet, und das Wechsellicht-Orientierungssignal und/oder das Wechsellicht-Erhaltungssignal vorzugsweise einen Winkelbereich von 0,1° bis 3° in vertikaler und in horizontaler Richtung aufweist bzw. aufweisen.

14. Verfahren nach einem der Ansprüche 1 bis 13, **dadurch gekennzeichnet, dass** das Wechsellicht-Orientierungssignal und das Wechsellicht-Erhaltungssignal durch Pulsweitermodulation von mindestens einer LED-Lichtquelle, die in den Scheinwerfermitteln bzw. in der mindestens einen Frontleuchte angeordnet ist, erzeugt werden.

15. Fahrassistenzsystem (601) für ein Kraftfahrzeug (600) zum Ausführen des Verfahrens zur Steuerung und/oder zum Trainieren des Fahrverhaltens eines Fahrers (602) nach einem der Ansprüche 1 bis 19, umfassend:
(i) mindestens eine den Fahrer betreffende Blickerfassungs-Einrichtung (603) (Eyetrackingeinrichtung), die zum kontinuierlichen Erfassen zumindest eines Blickparameters des Fahrers eingerichtet ist, wobei der Blickparameter aus der Gruppe bestehend aus einer Blickrichtung, einer Blickbewegung und/oder einer Blickdauer ausgewählt ist,
(ii) Scheinwerfermittel (606a, 606b), die dazu eingerichtet sind, zumindest eine vorgebbare Lichtverteilung vor dem Kraftfahrzeug (600) zu erzeugen, oder mindestens eine Frontleuchte (604),
(iii) Mittel zum Erfassen der aktuellen Verkehrssituation (605), und
(iv) eine Steuerungs- und Auswerteeinrichtung, welche dazu eingerichtet ist, die Scheinwerfermittel (606a, 606b) oder die mindestens eine Frontleuchte (604) zu steuern,
**dadurch gekennzeichnet, dass**
die Steuerungs- und Auswerteeinrichtung dazu eingerichtet ist, die folgenden Schritte durchzuführen, wenn die erfassten Blickparameter und/oder die Verkehrssituation im Vergleich zu abgespeicherten diesbezüglichen Normalwerten Abweichungen über vorgebbare Grenzen hinaus aufweisen:
Übermitteln eines ersten Steuerungssignals an die Scheinwerfermittel (606a, 606b) oder die mindestens eine Frontleuchte (604), um ein Wechsellicht-Orientierungssignal, welches in einer vorgebbaren Position vor dem Kraftfahrzeug (600) definiert ist und sich in seiner Amplitude der Lichtintensität für den Fahrer deutlich von einer aktuell vor dem Kraftfahrzeug befindlichen lokalen Umgebungshelligkeit abhebt,
Aufrechterhalten des Wechsellicht-Orientierungssignals, solange, bis eine Änderung zumindest eines Blickparameters des Fahrers durch die Blickerfassungs-Einrichtung (603) erfasst wird, wodurch ein Anzeichen für die Zuwendung der Aufmerksamkeit des Fahrers auf das Wechsellicht-Orientierungssignal festgestellt wird, und
Übermitteln eines zweiten Steuerungssignals an die Scheinwerfermittel (606a, 606b) oder die zumindest eine Frontleuchte (604), um das Wechsellicht-Orientierungssignals in ein Wechsellicht-Erhaltungssignal durch Herabsetzen der Frequenz sowie der Amplitude des Wechsellicht-Orientierungssignals auf einen für den auf das Orientierungslichtsignal aufmerksam gewordenen Fahrer erkennbaren Erhaltungswert umzuwandeln.

16. Fahrassistenzsystem nach Anspruch 15, **dadurch gekennzeichnet, dass** die Mittel zum Erfassen der aktuellen Verkehrssituation (605) Umfeldsensorikmittel zum Erfassen von Fahrzeugumfelddaten umfassen und dass die Steuerungs- und Auswerteeinrichtung dazu eingerichtet ist, die Fahrzeugumfelddaten zu empfangen, auszuwerten und in der Steuerung der Scheinwerfermittel zu berücksichtigen, wobei die Umfeldsensorikmittel vorzugsweise zumindest eines der folgenden Sensormittel umfassen: optische Sensormittel, Ultraschallsensormittel, Radarsensormittel, Lidar-Sensormittel, Infrarotsensormittel, und wobei die optischen Sensormittel vorzugsweise zumindest ein Kamerasystem mit zumindest einer Kamera umfassen.

## Claims

1. Method for controlling and/or training the driving behavior of a driver when driving a motor vehicle, the motor vehicle having: (i) at least one gaze detection device relating to the driver, (ii) headlight means which are adapted to generate at least one predeterminable light distribution in front of the motor vehicle or at least one front light, and (iii) means for detecting the current traffic situation, the method comprising:
continuously detecting at least one gaze parameter of the driver by means of the at least one gaze detection device (eye tracking device), wherein the gaze parameter is selected from the group consisting of a gaze direction, a gaze movement and a gaze duration of the driver as well as a combination of these gaze parameters,
and at least one warning signal is triggered for the driver if the recorded gaze parameters and/or the traffic situation exhibit deviations beyond predeterminable limits in comparison with stored normal values relating thereto,
the method being **characterized in that**
the advisory signal is transmitted to the driver by means of the "attractive flicker" method, which comprises the following steps:
generating, by means of the headlight means or the at least one front light, an alternating light orientation signal which is defined in a predeterminable position in the traffic space in front of the motor vehicle and whose amplitude of light intensity for the driver differs significantly from a local ambient brightness currently located in front of the motor vehicle,
maintaining the alternating light orientation signal until a change in at least one gaze parameter of the driver is detected by the gaze detection device, thereby detecting an indication that the driver's attention has been turned to the alternating light orientation signal, and
converting the alternating light orientation signal into an alternating light sustain signal by reducing the frequency as well as the amplitude of the alternating light orientation signal to a sustain value detectable by the driver attentive to the orientation light signal.

2. Method according to claim 1, **characterized in that** at least one saccade is detected by means of the at least one gaze detection device.

3. Method according to claim 1 or 2, **characterized in that** the sustain value of the alternating light sustain signal is superimposed on a DC component of the local ambient brightness currently located in front of the motor vehicle.

4. Method according to one of claims 1 to 3, **characterized in that** the value of the brightness difference of the alternating light orientation signal with respect to the local ambient brightness currently located in front of the motor vehicle is at least 8%, preferably between 8% and 15%.

5. Method according to one of claims 1 to 4, **characterized in that** the value of the brightness difference of the alternating light sustain signal to the local ambient brightness currently located in front of the motor vehicle is at least 5%, preferably 5-10%.

6. Method according to any one of claims 1 to 5, **characterized in that** the alternating light orientation signal has a frequency of 10 Hz to 100 Hz.

7. Method according to any one of claims 1 to 6, **characterized in that** the alternating light orientation signal is an on/off blink signal having a frequency of about 20 Hz.

8. Method according to any one of claims 1 to 4, **characterized in that** the alternating light sustain signal has a frequency of 0.5 Hz to 50 Hz.

9. Method according to any one of claims 1 to 8, **characterized in that** the alternating light sustain signal is an on/off flash signal having a frequency of about 2 Hz.

10. Method according to any one of claims 1 to 7, **characterized in that** the alternating light orientation signal lasts for at least 60 milliseconds and preferably up to 500 milliseconds.

11. Method according to any one of claims 1 to 10, **characterized in that** the alternating light orientation signal is adapted if no change in the at least one gaze parameter of the driver is detected by the gaze detection device within a predeterminable period of time and the adaptation of the alternating light orientation signal is performed by a change in the amplitude of the light intensity, the frequency, the duration or the angular range of the alternating light orientation signal or a combination thereof.

12. Method according to any one of claims 1 to 11, **characterized in that** further warning signals are transmitted to the driver, which are selected from subtle optical light stimuli for influencing the driver's direction of gaze (Subtle Gaze Direction (SGD) technology) and/or flashing light signals and/or acoustic signals.

13. Method according to one of claims 1 to 12, **characterized in that** the alternating light orientation signal and/or the alternating light sustain signal is/are realized as an animated light symbol, which optionally differs in color from the currently generated light distribution, and the alternating light orientation signal and/or the alternating light sustain signal preferably has/have an angular range of 0.1° to 3° in the vertical and in the horizontal direction.

14. Method according to any one of claims 1 to 13, **characterized in that** the alternating light orientation signal and the alternating light sustain signal are generated by pulse width modulation of at least one LED light source arranged in the headlight means and in the at least one front light, respectively.

15. Driving assistance system (601) for a motor vehicle (600) for performing the method of controlling and/or training the driving behavior of a driver (602) according to any one of claims 1 to 19, comprising:
(i) at least one gaze detection means (603) relating to the driver (eye tracking means), which is arranged to continuously detect at least one gaze parameter of the driver, wherein the gaze parameter is selected from the group consisting of a gaze direction, a gaze movement and/or a gaze duration,
(ii) headlight means (606a, 606b) adapted to generate at least one predeterminable light distribution in front of the motor vehicle (600), or at least one front light (604),
(iii) means for detecting the current traffic situation (605), and
(iv) a control and evaluation device which is adapted to control the headlight means (606a, 606b) or the at least one front light (604),
**characterized in that**
the control and evaluation device is adapted to carry out the following steps if the detected viewing parameters and/or the traffic situation exhibit deviations beyond predeterminable limits in comparison with stored normal values relating thereto:
transmitting a first control signal to the headlight means (606a, 606b) or the at least one front light (604) in order to generate an alternating light orientation signal which is defined in a predeterminable position in front of the motor vehicle (600) and whose amplitude of the light intensity for the driver clearly differs from a local ambient brightness currently located in front of the motor vehicle,
maintaining the alternating light orientation signal until a change in at least one gaze parameter of the driver is detected by the gaze detection device (603), thereby detecting an indication of the turning of the driver's attention to the alternating light orientation signal, and
transmitting a second control signal to the headlight means (606a, 606b) or the at least one front light (604) to convert the alternating light orientation signal into an alternating light sustain signal by reducing the frequency as well as the amplitude of the alternating light orientation signal to a sustain value detectable by the driver attentive to the orientation light signal.

16. Driving assistance system according to claim 15, **characterized in that** the means for detecting the current traffic situation (605) comprise environment sensor means for detecting vehicle environment data, and **in that** the control and evaluation device is adapted to receive the vehicle environment data, evaluate it and take it into account in the control of the headlight means, the environment sensor means preferably comprising at least one of the following sensor means: optical sensor means, ultrasonic sensor means, radar sensor means, lidar sensor means, infrared sensor means, and wherein the optical sensor means preferably comprise at least one camera system with at least one camera.

## Revendications

1. Procédé de contrôle et/ou de formation du comportement de conduite d'un conducteur lors de la conduite d'un véhicule à moteur, le véhicule à moteur ayant : (i) au moins un dispositif de détection du regard relatif au conducteur, (ii) des moyens de phare qui sont agencés pour générer au moins une distribution de lumière prédéterminable devant le véhicule automobile ou au moins un phare avant, et (iii) des moyens de détection de la situation actuelle de la circulation, le procédé comprenant :
détecter en continu au moins un paramètre du regard du conducteur au moyen de l'au moins un dispositif de détection du regard (dispositif de suivi du regard), le paramètre du regard étant sélectionné dans le groupe constitué par une direction du regard, un mouvement du regard et une durée du regard du conducteur, et une combinaison de ces paramètres du regard,
et au moins un signal d'avertissement pour le conducteur est déclenché si les paramètres du regard enregistrés et/ou la situation de la circulation présentent des écarts au-delà de limites prédéterminées par rapport aux valeurs normales mémorisées y afférentes,
le procédé étant **caractérisé en ce que**
le signal d'avertissement est transmis au conducteur au moyen du procédé du « attractive flicker », qui comprend les étapes suivantes :
générer, au moyen des moyens de phare ou d'au moins un phare avant, un signal d'orientation de lumière alternante qui est défini dans une position prédéfinie dans l'espace de la circulation devant le véhicule à moteur et dont l'amplitude de l'intensité lumineuse pour le conducteur diffère sensiblement d'une luminosité ambiante locale actuellement située devant le véhicule à moteur,
maintenir le signal d'orientation de lumière alternante jusqu'à ce qu'un changement d'au moins un paramètre du regard du conducteur soit détecté par le dispositif de détection du regard, détectant ainsi une indication de l'attention portée par le conducteur au signal d'orientation de lumière alternante, et
convertir le signal d'orientation de lumière alternante en un signal de maintien de lumière alternante en réduisant la fréquence ainsi que l'amplitude du signal d'orientation de lumière alternante à une valeur de maintien détectable par le conducteur attentif au signal de la lumière d'orientation.

2. Procédé selon la revendication 1, **caractérisé en ce qu'**au moins une saccade est détectée au moyen de l'au moins un dispositif de détection du regard.

3. Procédé selon la revendication 1 ou 2, **caractérisé en ce que** la valeur de maintien du signal de maintien de lumière alternante est superposée à une composante continue de la luminosité ambiante locale actuellement devant le véhicule automobile.

4. Procédé selon l'une quelconque des revendications 1 à 3, **caractérisé en ce que** la valeur de la différence de luminosité du signal d'orientation de lumière alternante par rapport à la luminosité ambiante locale actuellement située devant le véhicule automobile est d'au moins 8 %, de préférence entre 8 % et 15 %.

5. Procédé selon l'une quelconque des revendications 1 à 4, **caractérisé en ce que** la valeur de la différence de luminosité du signal de maintien de lumière alternante par rapport à la luminosité ambiante locale actuellement située devant le véhicule automobile est d'au moins 5 %, de préférence de 5 à 10 %.

6. Procédé selon l'une quelconque des revendications 1 à 5, **caractérisé en ce que** le signal d'orientation de lumière alternante a une fréquence de 10 Hz à 100 Hz.

7. Procédé selon l'une quelconque des revendications 1 à 6, **caractérisé en ce que** le signal d'orientation de lumière alternante est un signal clignotant marche/arrêt ayant une fréquence d'environ 20 Hz.

8. Procédé selon l'une quelconque des revendications 1 à 4, **caractérisé en ce que** le signal de lumière alternante de maintien a une fréquence de 0,5 Hz à 50 Hz.

9. Procédé selon l'une quelconque des revendications 1 à 8, **caractérisé en ce que** le signal de maintien de lumière alternante est un signal clignotant marche/arrêt ayant une fréquence d'environ 2 Hz.

10. Procédé selon l'une quelconque des revendications 1 à 7, **caractérisé en ce que** le signal d'orientation de lumière alternante dure au moins 60 ms et de préférence jusqu'à 500 ms.

11. Procédé selon l'une quelconque des revendications 1 à 10, **caractérisé en ce que** le signal d'orientation de lumière alternante est adapté si aucune modification d'au moins un paramètre du regard du conducteur n'est détectée par le dispositif de détection du regard dans une période de temps prédéterminable, et l'adaptation du signal d'orientation de lumière alternante est effectuée en modifiant l'amplitude de l'intensité lumineuse, la fréquence, la durée ou la plage angulaire du signal d'orientation de lumière alternante ou une combinaison de celles-ci.

12. Procédé selon l'une quelconque des revendications 1 à 11, **caractérisé en ce que** d'autres signaux d'indication sont transmis au conducteur, sélectionnés parmi des stimuli lumineux optiques subtils pour influencer la direction du regard du conducteur (technologie de la direction subtile du regard (SGD)) et/ou des signaux lumineux clignotants et/ou des signaux acoustiques.

13. Procédé selon l'une quelconque des revendications 1 à 12, **caractérisé en ce que** le signal d'orientation et/ou le signal de maintien de lumière alternante est/sont réalisé(s) comme symbole lumineux animé, qui se distingue éventuellement par sa couleur de la distribution de lumière actuellement générée, et de préférence le signal d'orientation et/ou le signal de maintien de lumière alternante comportant dans une plage angulaire de 0,1° à 3° en direction verticale et horizontale.

14. Procédé selon l'une quelconque des revendications 1 à 13, **caractérisé en ce que** le signal d'orientation de lumière alternante et le signal de maintien de lumière alternante sont générés par modulation de largeur d'impulsion d'au moins une source de lumière à LED disposée dans les moyens de phare ou dans le au moins un phare avant, respectivement.

15. Système d'assistance à la conduite (601) pour un véhicule à moteur (600) pour exécuter le procédé de contrôle et/ou de formation du comportement de conduite d'un conducteur (602) selon l'une quelconque des revendications 1 à 19, comportant :
(i) au moins un moyen de détection du regard (603) relatif au conducteur (dispositif de suivi du regard), qui est agencé pour détecter en continu au moins un paramètre du regard du conducteur, dans lequel le paramètre du regard est sélectionné dans le groupe constitué par une direction du regard, un mouvement du regard et/ou une durée du regard,
(ii) des moyens de phare (606a, 606b) agencés pour produire au moins une distribution lumineuse prédéterminable à l'avant du véhicule à moteur (600), ou au moins un phare avant (604),
(iii) des moyens de détection de la situation actuelle de la circulation (605), et
(iv) un dispositif de contrôle et d'évaluation qui est conçu pour commander les moyens de phare (606a, 606b) ou au moins un phare avant (604),
**caractérisé en ce que**
le dispositif de contrôle et d'évaluation est configuré pour effectuer les étapes suivantes si les paramètres de visualisation détectés et/ou la situation de la circulation présentent des écarts au-delà de limites prédéterminés par rapport aux valeurs normales enregistrées y afférentes :
transmettre un premier signal de commande aux moyens de phare (606a, 606b) ou audit au moins un phare avant (604) afin de générer un signal d'orientation de lumière alternante qui est défini dans une position prédéfinie devant le véhicule automobile (600) et dont l'amplitude de l'intensité lumineuse pour le conducteur diffère sensiblement d'une luminosité ambiante locale actuellement située devant le véhicule automobile,
maintenir le signal d'orientation de lumière alternante jusqu'à ce qu'un changement d'au moins un paramètre du regard du conducteur soit détecté par le dispositif de détection du regard (603), détectant ainsi une indication de l'attention portée par le conducteur au signal d'orientation de lumière alternante, et
transmettre un second signal de commande aux moyens de phare (606a, 606b) ou audit au moins un phare avant (604) pour convertir le signal d'orientation de lumière alternante en un signal de maintien de lumière alternante en réduisant la fréquence ainsi que l'amplitude du signal d'orientation de lumière alternante à une valeur de maintien détectable par le conducteur attentif au signal de la lumière d'orientation.

16. Système d'assistance à la conduite selon la revendication 15, **caractérisé en ce que** les moyens de détection de la situation actuelle de la circulation (605) comprennent des moyens de capteurs d'environnement pour détecter les données d'environnement du véhicule, et **en ce que** le dispositif de commande et d'évaluation est conçu pour recevoir les données d'environnement du véhicule, les évaluer et les prendre en compte dans la commande des moyens de phare, les moyens de capteurs d'environnement comportant de préférence au moins l'un des moyens de capteurs suivants : des moyens de capteurs optiques, des moyens de capteurs ultrasoniques, des moyens de capteurs radar, des moyens de capteurs lidar, des moyens de capteurs infrarouges ; et les moyens de capteurs optiques comportant de préférence au moins un système de caméra avec au moins une caméra.
